# FASCICULE DE BREVET EUROPEEN

(11) **EP 3 166 586 B1**
(45) Date de publication et mention de la délivrance du brevet: **15.04.2020**
(21) Numéro de dépôt: 15733448.3
(22) Date de dépôt: 01.07.2015
(51) Int. Cl.: A61K 8/60, A61Q 13/00, A61Q 15/00, A61K 8/73, A61K 8/92, A61K 8/11

(54) **HUILE ANHYDRE À BASE DE PARTICULES D'ENCAPSULATION D'UN AGENT BÉNÉFIQUE**
WASSERFREIES ÖL BEZOGEN AUF FREISETZUNGSPARTIKELN ENTHALTEND PFLEGEMITTEL
ANHYDROUS OIL BASED ON DELIVERY PARTICLES CONTAINING A BENEFIT AGENT

(30) Priorité: 09.07.2014 FR 1456633
(43) Date de publication de la demande: 17.05.2017
(73) Titulaire: L'Oréal, 75008 Paris (FR)
(72) Inventeur: MALLE, Gérard, 77580 Villiers S/Morin (FR); LUUKAS, Tiina, 93270 Sevran (FR); BARA, Isabelle, 94210 La Varenne St Hilaire (FR)
(74) Mandataire: L'Oreal
(86) Numéro de dépôt international: PCT/EP2015/065008
(87) Numéro de publication internationale: WO 2016/005249

(56) Documents cités:
- DE-A1-102008 035 013
- JP-A- 2008 156 236
- US-A- 5 508 259
- US-A1- 2004 029 750
- US-B1- 6 200 949
- MARTIN ET AL: "Encapsulation and Co-Precipitation Processes with Supercritical Fluids:Applications with Essential Oils", THE OPEN CHEMICAL ENGINEERING JOURNAL,, vol. 4, 1 janvier 2010 (2010-01-01), pages 31-41, XP002737531,

## Description

La présente invention concerne une composition anhydre sous forme d'huile comprenant :
1) au moins des particules comprenant un cœur contenant au moins un agent bénéfique et une enveloppe entourant le cœur ; ladite enveloppe comprenant au moins un polysaccharide modifié hydrophobe choisi parmi les C₅-C₂₀-alcényl succinates d'amidon et au moins un glucide hydrosoluble choisi parmi les maltodextrines ayant un Dextrose Équivalent allant de 4 à 20;lesdites particules présentant simultanément une densité de poudre versée allant de 300,0 g/l à 600,0 g/l et une densité absolue supérieure à 1,0; lesdites particules étant sphériques et ayant un diamètre moyen en nombre allant de 1 à 30µm et un diamètre moyen en volume allant de 5 à 150µm; et
2) une phase huileuse.

L'invention concerne en outre un procédé cosmétique de soin et/ou de maquillage d'une matière kératinique consistant à appliquer sur la surface de ladite matière kératinique humaine un produit de consommation comprenant une composition telle que définie précédemment.

La présente invention concerne également un procédé cosmétique de traitement des odeurs corporelles et éventuellement de la transpiration humaine consistant à appliquer sur la surface de la matière kératinique une composition telle que définie précédemment comprenant au moins un actif déodorant et/ou un actif anti-transpirant.

De nombreuses formes galéniques cosmétiques permettent de délivrer des agents bénéfiques notamment dans l'industrie cosmétique, dans l'industrie pharmaceutique, dans la parfumerie; dans les produits à usage vétérinaire notamment les produits d'hygiène et/ou de soin des animaux; dans les produits d'entretien ménager comme les produits pour le soin et/ou le nettoyage du linge; dans les produits d'entretien des appareils électroménagers; dans les produits d'entretien des sols, carrelages, bois etc; dans les produits sanitaires; dans les produits d'entretien des matières textiles; dans les produits d'entretien dans la maroquinerie comme les chaussures, les semelles; dans les produits issus de l'industrie agro-alimentaire; dans les produits issus de l'agriculture; dans les produits phytosanitaires; dans les peintures; dans les encres; dans les produits d'entretien dans l'industrie automobile.

Parmi celles-ci, les compositions sous forme d'huiles constituent une catégorie de produits appréciée par les consommateurs pour leur facilité d'étalement et d'application et notamment en cosmétique. Elles sont en particulier utilisées dans le domaine des produits solaires mais peuvent également être valorisées dans des produits de maquillage ou de soin des matières kératiniques comme la peau telles que les huiles de soin pour le corps comme les huiles de massage ou le visage ou les lèvres, les huiles auto-bronzantes ou les huiles pour le soin et/ou le conditionnement et/ou le coiffage des cheveux telles que les huiles de coiffage.

Le but de la présente invention est de proposer de nouvelles compositions cosmétiques de type huile anhydre comprenant au moins un agent bénéfique encapsulé dans des particules qui seraient étanches à l'abri de l'humidité c'est-à-dire inodores si l'actif est un parfum
- lesdites particules présentant une densité de poudre versée faible pour faciliter leur formulation et conserver une texture légère et douce
- lesdites particules devant également être compatibles avec les ingrédients habituels de ces formulations et suffisamment résistantes pour pouvoir être formulées en dispersion huileuse sans être endommagées
- ledit agent bénéfique contenu dans les particules pouvant être libéré de façon quasi immédiate, progressive et répétable sur la peau, le cheveu et les phanères au contact de l'eau

On sait qu'il existe une nécessité dans de nombreux domaines industriels, de protéger un certain nombre de molécules fragiles ou volatiles et de contrôler leur relargage vers un milieu extérieur.

Un des moyens permettant d'atteindre un tel but est leur encapsulation. Cette encapsulation a pour objectif de diminuer l'évaporation et le transfert vers l'environnement de la matière active, soit au cours du stockage, soit au cours de l'élaboration des produits ou encore au cours de leur utilisation. Elle peut également permettre de rendre le matériau plus facile d'utilisation en le diluant et en favorisant sa répartition homogène au sein du support.

La micro-encapsulation regroupe l'ensemble des technologies permettant l'enrobage ou le piégeage de principes actifs sous forme solide, liquide, ou gazeuse au sein de particules individualisées dont la taille s'échelonne entre quelques microns et quelques millimètres. Si ces microparticules sont creuses (vésiculaires) on parle de microcapsules, si elles sont pleines (matricielles) on parle de microsphères. Leur taille varie de 1µm à plus de 1000µm. Ces microparticules peuvent être biodégradables ou non et peuvent contenir entre 5 et 90% (en masse) de substance active.

Les substances actives encapsulées sont d'origines très variées: principes actifs pharmaceutiques, cosmétiques, additifs alimentaires, produits phytosanitaires, essences parfumées, micro-organismes, cellules, ou encore catalyseurs de réaction chimique...

Tout l'intérêt des microparticules d'encapsulation réside dans la présence d'une membrane polymérique, qui isole et protège le contenu du milieu extérieur. Selon les cas, la membrane sera détruite lors de l'utilisation pour libérer son contenu (exemple : encarts publicitaires "scratch and sniff" libérant le parfum lorsqu'on écrase les microcapsules), ou bien la membrane restera présente tout le long de la libération du contenu, dont elle contrôlera la vitesse de diffusion (exemple: encapsulation de médicaments pour libération ralentie).

Les matériaux d'enrobage sont généralement des polymères d'origine naturelle ou synthétique, hydrophobes ou hydrophiles, ou bien des lipides (JP 2008 156236, DE 10 2008 035013).

Les principaux procédés pour réaliser l'encapsulation de substances dans des microparticules sont la polymérisation interfaciale, la réticulation interfaciale, l'émulsion suivie d'une évaporation ou d'une extraction du solvant, la double émulsion évaporation/extraction de solvant, le spray-drying, le prilling, la coacervation.

Dans le brevet US 5 508 259, on a proposé des compositions parfumantes non aqueuses, comprenant des parfums encapsulés dans des particules solubles dans l'eau. Lesdites capsules sont obtenues par des techniques d'encapsulation conventionnelles et en particulier le spray-drying d'une émulsion constituée d'un substrat solide filmogène en combinaison avec un agent émulsionnant et un mélange d'ingrédients de parfumerie. Le substrat solide filmogène est notamment choisi parmi l'acétate de polyvinyle, l'alcool polyvinylique, des dextrines, de l'amidon naturel ou modifié, les gommes végétales, les pectines, les xanthanes, les alginates, carraghénanes ou encore les dérivés de cellulose tels que par exemple la carboxyméthylcellulose, la méthylcellulose ou l'hydroxyéthylcellulose. L'émulsion est ensuite déshydratée par un procédé classique d'atomisation (spray-drying), qui consiste, tel que décrit à l'exemple 1, à la pulvériser en fines gouttelettes dans un atomiseur avec un débit de 50kg/h et une pression de 0,45 bars, au contact d'un courant d'air de 320m³/h chauffé à 350°C afin d'évaporer l'eau, ce qui permet d'obtenir une poudre fine avec un diamètre de particules compris entre 20 et 80microns et contenant 20% en poids de parfum.

Cependant, on a remarqué que les particules obtenues par ce procédé étaient fortement odorantes à l'état sec à cause de la présence de parfum libre (non encapsulé), qu'elles étaient principalement constituées d'agglomérats pouvant nuire à l'homogénéité du produit et gêner la bonne application du produit et ne possédaient pas les caractéristiques de densité adaptées à l'objectif de l'invention.

Dans le brevet US 6 200 949, on a décrit également un procédé de formation d'une matière particulaire contenant un parfum hydrophile comprenant les étapes successives consistant à former une émulsion aqueuse de parfum contenant 40 à 60% en poids d'eau, 3 à 30% en poids de maltodextrine, 10 à 40% en poids d'amidon modifié hydrophobe, puis à la sécher par pulvérisation dans un atomiseur (courant d'air de 420m³/h chauffé à 204°C de sorte que les particules sont formées avec une taille moyenne d'environ 3 à environ 10 microns et une teneur en parfum de 15 à 50% en poids.

Cependant, les particules obtenues par ce procédé sont fortement odorantes à l'état sec à cause de la présence de parfum libre (non encapsulé), sont principalement constituées d'agglomérats, peuvent nuire à l'homogénéité du produit et ne possèdent pas les caractéristiques de densité adaptées à l'objectif de l'invention.

Il est évidemment très important de pouvoir disposer de particules étanches qui ne libèrent leur contenu qu'à la demande (en réponse à l'humidité ambiante, notamment dans les zones climatiques humides, en réponse à la transpiration corporelle, au shampooing ou sous la douche, etc...), d'une part pour garantir la protection dans le temps de l'actif encapsulé surtout s'il est fragile et/ou volatil et d'autre part pour éviter les interactions avec les autres ingrédients de la formule. Lorsque l'agent bénéfique encapsulé est un ingrédient de parfumerie et/ou un parfum complet, il est d'autant plus important que l'encapsulation soit totale, ce qui conduit à des particules inodores en formules anhydres permettant au formulateur de les associer ou non avec n'importe quel un parfum libre de son choix (identique ou différent) sans risque d'interactions ou de perturbation de la note parfumée choisie.

Dans le brevet EP1917098B1, on a proposé un procédé de préparation de produits d'encapsulation par précipitation, lequel procédé emploie :
- une émulsion pouvant être pompée comprenant (i) une phase continue contenant un solvant et un soluté formant une matrice dissout dans ledit solvant et (ii) une phase dispersée ;
- un extracteur comprenant un gaz supercritique, sous-critique ou liquéfié ;
   ledit solvant étant sensiblement plus soluble dans l'extracteur que ledit soluté formant une matrice et ledit procédé comprenant les étapes successives consistant à :
   a. combiner l'émulsion pouvant être pompée avec l'extracteur dans des conditions de mélange ;
   b. permettre la formation de produits d'encapsulation particulaires dans lesquels la phase dispersée est imbriquée dans une matrice solide du soluté formant une matrice ;
   c. collecter les produits d'encapsulation et les séparer de l'extracteur.

Il est indiqué que ce procédé peut être utilisé dans les industries pharmaceutique et agroalimentaire ainsi-que dans les domaines de l'agriculture, du « coating », des adhésifs et des catalyseurs. Il peut en particulier être utilisé pour encapsuler des actifs pharmaceutiques, des arômes, des enzymes, des colorants, des pesticides et des herbicides. Après d'importantes recherches, la demanderesse a découvert de façon surprenante et inattendue qu'il était possible d'atteindre les objectifs tels qu'énoncés précédemment en utilisant, dans une composition anhydre sous forme d'huile comprenant au moins une phase huileuse et des particules à libération d'agent bénéfique comprenant un cœur contenant au moins un agent bénéfique et une enveloppe entourant le cœur ; ladite enveloppe comprenant au moins un polysaccharide modifié hydrophobe choisi parmi les C₅-C₂₀-alcényl succinates d'amidon et au moins un glucide hydrosoluble choisi parmi les maltodextrines ayant un Dextrose Équivalent allant de 4 à 20; lesdites particules présentant simultanément une densité de poudre versée allant de 300,0 g/l à 600,0 g/l et une densité absolue supérieure à 1,0; et lesdites particules étant sphériques et ayant un diamètre moyen en nombre allant de 1 à 30µm et un diamètre moyen en volume allant de 5 à 150µm. Ces particules peuvent être en particulier obtenues par le procédé tel que décrit dans le brevet EP1917098B1 commenté précédemment.

Les particules à libération d'agent bénéfique conformes à la présente invention permettent d'encapsuler des ingrédients bénéfiques, en particulier fragiles, de façon complète (encapsulation totale) sans dégradation dans des capsules qui seraient suffisamment résistantes et étanches pour pouvoir être conservées sans altération à l'abri de l'humidité et pouvant être aisément formulées et rester stables dans des compositions anhydres sous forme d'huile. Ces mêmes particules dans ce type de composition présentent de préférence une morphologie sphérique et une densité de poudre versée très faible pour conserver la texture légère et douce; elles ont également la capacité de s'ouvrir en présence d'eau pour pouvoir libérer leur agent bénéfique de façon quasi immédiate, progressive et répétable sur la peau, le cheveu et les phanères au contact de l'eau. Cette découverte est à la base de la présente invention.

La présente invention concerne une composition anhydre sous forme d'huile comprenant :
1) au moins des particules comprenant un cœur contenant au moins un agent bénéfique et une enveloppe entourant le cœur ; ladite enveloppe comprenant au moins un polysaccharide modifié hydrophobe choisi parmi les C5-C20-alcényl succinates d'amidon et au moins un glucide hydrosoluble choisi parmi les maltodextrines ayant un Dextrose Équivalent allant de 4 à 20;lesdites particules présentant simultanément une densité de poudre versée allant de 300,0 g/l à 600,0 g/l et une densité absolue supérieure à 1,0; lesdites particules étant sphériques et ayant un diamètre moyen en nombre allant de 1 à 30µm et un diamètre moyen en volume allant de 5 à 150µm ; et
2) une phase huileuse.

L'invention concerne en outre un procédé cosmétique de soin et/ou de maquillage d'une matière kératinique consistant à appliquer sur la surface de ladite matière kératinique humaine un produit de consommation comprenant une composition telle que définie précédemment.

La présente invention concerne également un procédé cosmétique de traitement des odeurs corporelles et éventuellement de la transpiration humaine consistant à appliquer sur la surface de la matière kératinique une composition telle que définie précédemment comprenant au moins un actif déodorant et/ou un actif anti-transpirant.

De préférence, la composition comprend un milieu physiologiquement acceptable et, plus préférentiellement, cosmétiquement acceptable.

Les compositions sous forme d'huile anhydre selon l'invention peuvent être également utilisées dans d'autres domaines tels que les produits à usage vétérinaire notamment les produits d'hygiène et/ou de soin des animaux; les produits d'entretien ménager comme les produits pour le soin et/ou le nettoyage du linge; les produits d'entretien des appareils électroménagers; les produits d'entretien des sols, carrelages, bois etc; les produits sanitaires; les produits d'entretien des matières textiles; les produits d'entretien dans la maroquinerie comme les chaussures, les semelles; les produits issus de l'industrie agro-alimentaire; les produits issus de l'agriculture; les produits phytosanitaires; les peintures; les encres; les produits d'entretien dans l'industrie automobile.

### Définitions

Par «composition anhydre», on entend au sens de la présente invention, une composition présentant une teneur en eau inférieure à 5 % en poids, de préférence inférieure à 2% en poids et de manière encore plus préférée inférieure à 1% en poids par rapport au poids de ladite composition voire encore moins de 0,5% et notamment exempte d'eau. Dans cette définition, l'eau mentionnée inclut l'eau résiduelle apportée par les ingrédients mélangés

Par «composition sous forme d'huile», on entend une composition liquide à 25°C et pression atmosphérique (760mm de Hg) essentiellement constituée d'une phase huileuse.

Au sens de la présente demande, par « phase huileuse, on entend une phase huileuse liquide à température ambiante (25°C) et pression atmosphérique (760mm de Hg), composée d'un ou plusieurs corps gras liquides à température ambiante, appelés aussi huiles, compatibles entre eux.

Au sens de la présente invention, on entend désigner par «milieu physiologiquement acceptable», un milieu convenant à l'administration d'une composition par voie topique. Un milieu physiologiquement acceptable est un milieu sans odeur et/ou sans aspect désagréable, et qui est parfaitement compatible avec la voie d'administration topique.

Par "matière kératinique", on entend la peau, le cuir chevelu, les lèvres, et/ou les phanères tels que les ongles et les fibres kératiniques, telles que par exemple, les poils, les cils, les sourcils et les cheveux.

Par «composition cosmétique», on entend au sens de l'invention toute composition appliquée sur la surface d'une matière kératinique pour produire un effet non-thérapeutique d'hygiène, de soin, de conditionnement ou de maquillage contribuant à l'amélioration du bien-être et/ou à l'embellissement et/ou la modification de l'aspect de la matière kératinique sur laquelle on applique ladite composition.

Par «composition dermatologique», on entend au sens de l'invention toute composition appliquée sur la surface d'une matière kératinique pour prévenir et/ou traiter un désordre ou un dysfonctionnement de ladite matière kératinique.

Par «traitement cosmétique», on entend au sens de l'invention tout effet non-thérapeutique de parfumage, d'hygiène, de soin, de conditionnement ou de maquillage contribuant à l'amélioration du bien-être et/ou à l'embellissement et/ou la modification de l'aspect ou de l'odeur de la matière kératinique sur laquelle on applique ladite composition.

Par «produit de consommation», on entend tout produit fabriqué destiné à être utilisé ou consommé dans la forme où il est commercialisé et qui n'est pas destiné à une fabrication ou modification ultérieure. Sans que les exemples soient limitatifs, les produits de consommation selon l'invention peuvent être des produits cosmétiques incluant aussi bien des formulations cosmétiques pour le soin et/ou l'hygiène de la peau, des lèvres ou des cheveux; des produits dermatologiques; des produits de parfumerie; des produits pharmaceutiques; des produits à usage vétérinaire notamment des produits d'hygiène et/ou de soin des animaux; des produits d'entretien ménager comme les produits pour le soin et/ou le nettoyage du linge; des produits d'entretien des appareils électroménagers; des produits d'entretien des sols, carrelages, bois etc; des produits sanitaires; des produits d'entretien des matières textiles; des produits d'entretien dans la maroquinerie comme les chaussures, les semelles; des produits issus de l'industrie agro-alimentaire; des produits issus de l'agriculture; des produits phytosanitaires; des peintures; des encres; des produits d'entretien dans l'industrie automobile.

Par «agent bénéfique», on entend au sens de l'invention tout composé présent dans un produit de consommation produisant un effet bénéfique perçu par le consommateur lors de son utilisation et/ou obtenu sur le produit de consommation lui-même, ledit effet bénéfique pouvant être une amélioration sensorielle ou une modification notamment visuelle et/ou olfactive et/ou tactile, une amélioration du confort et /ou de la facilité d'application, un effet esthétique, un effet hygiénique, une sensation de propreté, un effet curatif et/ou prophylactique.

Par «particules comprenant un cœur contenant au moins un agent bénéfique», on entend une particule comprenant au moins un agent bénéfique immobilisé, capturé et/ou encapsulé dans la matrice d'un système d'encapsulation ou de piégeage; ledit agent bénéfique étant libéré vers l'extérieur au fur et à mesure de la détérioration du système d'encapsulation ou de piégeage lorsque sa dégradation se produit au contact d'un milieu avec lequel il va réagir ou sous l'effet d'un stimulus tel qu'un apport d'eau.

### Densité de poudre versée (ou densité apparente non tassée)

La détermination est effectuée à température ambiante (20-25°C) et dans les conditions normales atmosphériques (1 atmosphère) à l'aide d'une éprouvette de 100ml. L'éprouvette est pesée vide puis remplie d'un volume de 100ml de poudre versée, sans tassement. La différence de masse entre éprouvette vide et remplie de 100ml de poudre donne la densité de poudre versée.

### Densité absolue

### Principe de la mesure

La mesure consiste à déterminer le poids d'un échantillon du solide en poudre par simple pesée puis à mesurer le volume occupé par les particules de poudre en mesurant le volume de liquide déplacé par l'échantillon de poudre par immersion dans ce liquide. Le liquide choisi doit être peu volatil et ne doit pas être un solvant de la poudre. On choisit généralement le cyclohexane. Les mesures sont réalisées au moins deux fois.

Matériels : Une fiole jaugée de 10 ou 25ml et une balance de précision.
- m₁ est le poids de la fiole vide
- m₂ est le poids de la fiole remplie d'eau jusqu'au trait de jauge.
- m₃ est le poids de la fiole remplie de cyclohexane jusqu'au trait de jauge.
- m₄ est le poids de la fiole remplie environ au tiers de sa capacité par la poudre à analyser.

On remplit la fiole environ au tiers de sa capacité avec la poudre à analyser.

### Méthode

On complète la fiole, un peu en-dessous du trait de jauge avec du cyclohexane. Afin d'éliminer complètement l'air emprisonné dans la poudre on opère comme suit :
1) on traite la fiole dans un bac à ultra-sons pendant 5 minutes
2) on ajuste le niveau du cyclohexane jusqu'au trait de jauge
3) on traite la fiole dans un bac à ultra-sons pendant 2 minutes
4) les étapes 2 et 3 sont répétées si nécessaire jusqu'à ce que le niveau du cyclohexane n'évolue plus.
m₅ est le poids de la fiole ainsi remplie.

Le poids de poudre analysée est égal à m₄-m₁ (pour une bonne précision ce poids doit être supérieur à 2g). La densité de l'air étant très faible par rapport à celle du solide on admet que m₄ - m₁ est égal Le poids de cyclohexane correspondant au volume occupé par le solide (Vs) est égal à :
m₆ = (m₃ - m₁) - (m₅ - m₄) = ρ_{cyclo}.Vs où ρ_{cyclo} est la densité du cyclohexane à la température du laboratoire.

La densité absolue du solide constitutif de la poudre est égale à ρ_{cyclos} = (m₄ - m₁) /Vs = ρ_{cyclo}(m₄ - m₁) / m₆.

Si la densité du cyclohexane à la température du laboratoire n'est pas connue, on la détermine comme suit par rapport à celle de l'eau :
Soit Vf le volume jaugé de la fiole et ρₑₐᵤ la densité de l'eau à la température du laboratoire on a :
ρ_{cyclo} = (m₃ - m₁) / Vf et ρₑₐᵤ = (m₂ - m₁) / Vf
soit ρ_{cyclo} = ρₑₐᵤ (m₂ - m₁) / (m₃ - m₁)

La densité absolue du solide constitutif de la poudre est égale à :
ρₛ = [ρₑₐᵤ (m₄ - m₁) (m₂ - m₁)] / [m₆ (m₃ - m₁)].

### PARTICULES D'ENCAPSULATION

Les particules conformes à l'invention comprennent un cœur contenant au moins un agent bénéfique et une enveloppe entourant le cœur; ladite enveloppe comprenant au moins un polysaccharide modifié hydrophobe choisi parmi les C₅-C₂₀-alcényl succinates d'amidon et au moins un glucide hydrosoluble choisi parmi les maltodextrines ayant un Dextrose Équivalent allant de 4 à 20; lesdites particules présentant simultanément une densité de poudre versée allant de 300,0 g/l à 600,0 g/l et une densité absolue supérieure à 1,0; lesdites particules étant sphériques et ayant un diamètre moyen en nombre allant de 1 à 30µm et un diamètre moyen en volume allant de 5 à 150µm.

Les particules conformes à la présente invention sont sphériques.

Par «sphériques», on entend que la particule présente un indice de sphéricité, c'est-à-dire le rapport entre son plus grand diamètre et son plus petit diamètre, est inférieur à 1,2. Dans ce cas, de telles particules sont généralement appelées «capsules».

Par « taille moyenne » des particules on entend les paramètres D[4,3] et D[2,3] mesurés en voie sèche par diffraction laser à l'aide d'un granulomètre Microtrac S3500, les résultats étant exprimés sous la forme de distributions granulométriques en volume et en nombre donnant accès au diamètre moyen.

Les particules sphériques conformes à la présente invention présentent, de préférence, ainsi un diamètre moyen en nombre allant de 1 à 30µm, plus préférentiellement allant de 2 à 15µm et encore mieux de 5 à 10µm et un diamètre moyen en volume allant de 5 à 150µm, de préférence allant de 10 à 100µm et encore mieux de 20 à 80µm.

Les particules selon l'invention contenant l'agent bénéfique représentent, de préférence, de 0,1 à 60% en poids, de préférence 0,3 à 40% en poids et encore mieux de 0,5 à 20% en poids du poids total de la composition.

### POLYSACCHARIDE MODIFIE HYDROPHOBE

On entend par «polysaccharide modifié hydrophobe» tout polysaccharide modifié par voie chimique ou enzymatique et comportant au moins un groupe fonctionnel hydrophobe.

Les polysaccharides sont des macromolécules glucidiques formées par l'enchaînement d'un grand nombre de sucres élémentaires (oses) hydrophiles liés entre eux par des liaisons O-osidiques.

Les groupes fonctionnels hydrophobes de la présente invention sont des groupes hydrocarbonés (constitués essentiellement d'atomes de carbone et d'hydrogène) comprenant de préférence au moins 6 et encore mieux au moins 8 atomes de carbone alcènyles. Le nombre maximum d'atomes de carbone du groupe hydrocarboné est, de préférence, 24, plus préférentiellement 20, et encore plus préférentiellement 18. Les groupes hydrocarbonés hydrophobes peuvent être non substitués, par exemple constitués d'une simple longue chaine alkyle ou bien substitués par des groupes non réactifs comme des groupes aromatiques tels que des groupes aryles (ie: phényle) ou aralkyles (ie: benzyle) ou encore des groupes polaires tels que par exemple des carboxyles ou des hydroxyles.

Pour greffer le ou les groupe(s) fonctionnel(s) hydrophobe(s) sur les polysaccharides, on utilise généralement des dérivés des acides carboxyliques ou leurs dérivés (esters, halogénures d'acides, anhydrides).

Le polysaccharide modifié hydrophobe représente de préférence de 20 à 90% en poids, notamment 30 à 80% en poids, mieux 40 à 70% en poids, et encore mieux 40 à 60% en poids par rapport au poids total de l'enveloppe de la particule.

Les molécules d'amidons peuvent avoir comme origine botanique les céréales ou encore les tubercules. Ainsi, les amidons sont par exemple choisis parmi les amidons de maïs, de riz, de manioc, de tapioca, d'orge, de pomme de terre, de blé, de sorgho, de pois.

On entend par «amidon modifié hydrophobe» tout amidon modifié par réaction chimique ou enzymatique et comprenant au moins un groupe fonctionnel hydrophobe.

Les amidons modifiés hydrophobes conformes à l'invention sont choisis parmi les C₅-C₂₀ alcényl succinates d'amidon, plus particulièrement l'octényle succinate d'amidon sodique (E1450- CAS 66829-29-6 / 52906-93-1 / 70714-61-3), en particulier celui commercialisé par National Starch sous la dénomination CAPSUL®.

On peut également citer les références commerciales CAPSUL TA®, N-LOK®, N- LOK 1930®, HI-CAP 100®, PURITY GUM 1773®, PURITY GUM 2000 ® de National Starch, CLEARGUM CO® de la société Roquette et EMCAP®, EMTEX®, DELITEX de la société Cargill.

### GLUCIDE HYDROSOLUBLE

Par «glucide hydrosoluble » on entend un glucide ou polyol qui, introduit dans l'eau sans modification de pH à 25°C, à une concentration massique égale à 3%, permet l'obtention d'une solution macroscopiquement homogène et transparente, c'est à dire ayant une valeur de transmittance minimum de la lumière, à une longueur d'onde égale à 500nm, à travers un échantillon de 1cm d'épaisseur, d'au moins 80%, de préférence d'au moins 90%. Par «glucides» (encore appelés hydrates de carbone ou carbohydrates ou saccharides) on entend l'ensemble des sucres simples ou oses et leurs combinaisons ou osides.

Les glucides comprennent habituellement:
(1) les monosaccharides ou oses qui sont de deux types : les aldoses comprenant une fonction aldéhyde sur le premier carbone et les cétoses comprenant une fonction cétone sur le deuxième carbone. On les distingue aussi suivant le nombre d'atomes de carbone qu'ils possèdent.
(2) les oligosaccharides (ou oligosides) qui sont des oligomères d'oses ayant un enchaînement de 2 à 10 unités monosaccharides unies par des liaisons glycosidiques.
(3) Les polyholosides (ou polysaccharides ou polyosides) qui sont des polymères d'oses ayant un enchaînement de monosaccharides supérieur à 10 unités

### GLUCIDES HYDROSOLUBLES

Les glucides hydrosolubles de l'invention sont choisi parmi les maltodextrines ayant un Dextrose Équivalent allant de 4 à 20.

Les maltodextrines qui sont le résultat de l'hydrolyse d'un amidon (ie : blé, maïs) ou d'une fécule (ie : pomme de terre). Elles sont constituées de différents sucres (ie : glucose, maltose, maltotriose, oligosides et polyosides) directement issus de cette réaction, dans des proportions qui dépendent du degré de l'hydrolyse.

Ce degré est mesuré par « dextrose équivalent », ou D.E., le dextrose ou D-glucose étant le résultat d'une hydrolyse totale de l'amidon. Plus le D.E. est élevé, plus l'hydrolyse est poussée, et donc plus la proportion en sucres simples (à chaîne courte) composant la maltodextrine est élevée.

Les maltodextrines utilisées conformément à l'invention sont préférentiellement de D.E. allant de 12 à 20.

On utilisera de préférence les maltodextrines de pomme de terre ou de maïs telles que celles vendues sous les dénominations commerciales MD 20P® de AVEBE, MALDEX 120®, MALDEX 170®, MALDEX 190® de Tereos.

Parmi les glucides hydrosolubles conformes à l'invention, on choisi de D.E. allant de 4 à 20 et encore mieux les maltodextrines de D.E. allant de 12 à 20.

Le ou les glucides hydrosoluble(s) conformes à l'invention représentent de 10 à 80% en poids, de préférence de 15 à 70% en poids, plus préférentiellement de 20 à 65% en poids, et encore mieux de 40 à 60% en poids par rapport au poids total de l'enveloppe de la particule.

Selon l'invention, l'enveloppe des particules selon l'invention est constituée
a) d'au moins un C₅-C₂₀-alcényl succinate d'amidon et
b) d'au moins une maltodextrine de D.E. allant de 4 à 20 et de préférence allant de 12 à 20.

Selon une forme particulièrement préférée de l'invention, l'enveloppe des particules d'encapsulation est constituée
a) d'au moins un C₅-C₂₀-alcényl succinate d'amidon dans une quantité allant de 20 à 90% en poids, notamment 30 à 80% en poids, de préférence 40 à 70% et encore mieux 40 à 60% en poids, par rapport au poids total de l'enveloppe de la particule.
b) et d'au moins une maltodextrine de D.E. allant de 4 à 20 dans une quantité allant de 10 à 80% en poids, de préférence de 15 à 70% en poids, plus préférentiellement de 20 à 65% en poids, et encore mieux de 40 à 60% en poids par rapport au poids total de l'enveloppe de la particule.

### Procédé de préparation des particules à libération d'agent bénéfique

Les particules selon l'invention peuvent notamment être préparées selon le procédé décrit dans le brevet EP1917098B1 de FeyeCon.

Selon une forme particulière de l'invention, les particules sont obtenues selon un procédé comprenant au moins les étapes suivante :
- on prépare une solution aqueuse constituée du mélange du glucide hydrosoluble choisi parmi les maltodextrines de dextrose Équivalent allant de 4 à 20 et du polysaccharide modifié hydrophobe choisi parmi les C₅-C₂₀-alcényl succinates d'amidon puis on ajoute l'agent bénéfique et on agite de façon à former une émulsion; et
- on homogénéise ladite émulsion ainsi formée sous haute pression à une pression allant de 10 à 200 bars et plus préférentiellement de 20 à 200 bars; et
- on pulvérise, de préférence en continu, ladite émulsion dans une chambre de séchage; et
- l'eau est extraite pendant une durée, de préférence, ne dépassant pas 3 heures, et plus préférentiellement ne dépassant pas 30 minutes, avec un fluide sous pression tel que le dioxyde de carbone, de préférence à l'état supercritique, de préférence à une pression d'au moins 0,3XPc et à une température d'au moins Tc-60°C avec Pc correspondant à la pression critique du gaz et Tc la température critique du gaz, de façon à obtenir des particules, de préférence sphériques, de taille moyenne, de préférence allant de 1 à 150µm, plus préférentiellement allant de 2 à 100µm et encore mieux de 5 à 80µm.

### PHASE HUILEUSE

La composition comprend au moins une phase huileuse, notamment à raison de 70% à 99,9% en poids par rapport au poids, de préférence de 80%à 99,7% et encore mieux de 90% à 99,5% en poids par rapport au poids total de la composition.

La phase huileuse comprend généralement au moins une huile volatile et/ou une huile non volatile.

Par «huile», on entend un corps gras liquide à température ambiante (25°C) et pression atmosphérique (760mm de Hg soit 105Pa). L'huile peut être volatile ou non volatile.

Par « huile volatile », on entend au sens de l'invention une huile susceptible de s'évaporer au contact de la peau ou de la fibre kératinique en moins d'une heure, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,13Pa à 40 000Pa (10⁻³ à 300mm de Hg), en particulier allant de 1,3Pa à 13 000Pa (0,01 à 100mm de Hg), et plus particulièrement allant de 1,3Pa à 1300Pa (0,01 à 10mm de Hg).

Par «huile non volatile», on entend une huile restant sur la peau ou la fibre kératinique à température ambiante et pression atmosphérique au moins plusieurs heures et ayant notamment une pression de vapeur inférieure à 10⁻³mm de Hg (0,13Pa).

L'huile peut être choisie parmi toutes les huiles physiologiquement acceptables et en particulier cosmétiquement acceptables, notamment les huiles minérales, animales, végétales, synthétique ; en particulier les huiles hydrocarbonées et/ou siliconées et/ou fluorées volatiles ou non volatiles et leurs mélanges.

Plus précisément, par «huile hydrocarbonée», on entend une huile comportant principalement des atomes de carbone et d'hydrogène et éventuellement une ou plusieurs fonctions choisies parmi les fonctions hydroxyle, ester, éther, carboxylique. Généralement, l'huile présente une viscosité de 0,5 à 100 000mPa.s, de préférence de 50 à 50 000mPa.s et de préférence encore de 100 à 300 000mPa.s.

A titre d'exemple d'huile volatile utilisable dans l'invention, on peut citer:
- les huiles hydrocarbonées volatiles choisies parmi les huiles hydrocarbonées ayant de 8 à 16 atomes de carbones, et notamment les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars ou de Permetyls, les esters ramifiés en C₈-C₁₆, le néopentanoate d'iso-hexyle, et leurs mélanges. D'autres huiles hydrocarbonées volatiles comme les distillats de pétrole, notamment ceux vendus sous la dénomination Shell Soit par la société SHELL, peuvent aussi être utilisées ; les alcanes linéaires volatils comme ceux décrits dans la demande de brevet de la société Cognis DE10 2008 012 457.
- les silicones volatiles, comme par exemple les huiles de silicones linéaires ou cycliques volatiles, notamment celles ayant une viscosité ≤ 8 centistokes (8 10⁻⁶m²/s), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane
- et leurs mélanges.

On peut également citer les huiles linéaires alkyltrisiloxanes volatiles de formule générale (I): 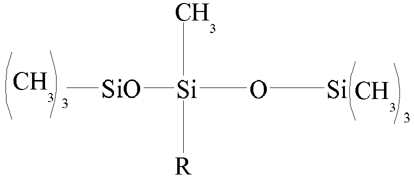 où R représente un groupe alkyle comprenant de 2 à 4 atomes de carbone et dont un ou plusieurs atomes d'hydrogène peuvent être substitués par un atome de fluor ou de chlore.

Parmi les huiles de formule générale (I), on peut citer:
le 3-butyl 1,1,1,3,5,5,5-heptaméthyl trisiloxane,
le 3-propyl 1,1,1,3,5,5,5-heptaméthyl trisiloxane, et
le 3-éthyl 1,1,1,3,5,5,5-heptaméthyl trisiloxane correspondant aux huiles de formule (I) pour lesquelles R est respectivement un groupe butyle, un groupe propyle ou un groupe éthyle.

A titre d'exemple d'huile non volatile utilisable dans l'invention, on peut citer:
- les huiles hydrocarbonées d'origine animale telles que le perhydrosqualène;
- les huiles hydrocarbonées végétales telles que les triglycérides liquides d'acides gras de 4 à 24 atomes de carbone comme les triglycérides des acides heptanoïque ou octanoïque ou encore les huiles les huiles de germe de blé, d'olive, l'huile d'amande douce, de palme, de colza, de coton, de luzerne, de pavot, de potimarron, de courge, de cassis, d'onagre, de millet, d'orge, de quinoa, de seigle, de carthame, de bancoulier, de passiflore, de rosier muscat, de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel;
- les hydrocarbures linéaires ou ramifiés, d'origine minérale ou synthétique tels que les huiles de paraffine et leurs dérivés, la vaseline, les polydécènes, les polybutènes, le polyisobutène hydrogéné tel que le Parleam, le squalane ;
- les éthers de synthèse ayant de 10 à 40 atomes de carbone;
- les esters de synthèse notamment d'acides gras comme les huiles de formule R₁COOR₂ dans laquelle R₁ représente le reste d'un acide gras supérieur linéaire ou ramifié comportant de 1 à 40 atomes de carbone et R₂ représente une chaîne hydrocarbonée notamment ramifiée contenant de 1 à 40 atomes de carbone avec R₁ + R₂ ≥ 10 comme par exemple l'huile de Purcellin (octanoate de cétostéaryle), l'isononanoate d'isononyle, le myristate d'isopropyle, le palmitate d'isopropyle, les benzoates d'alcool en C₁₂ à C₁₅, le laurate d'hexyle, l'adipate de diisopropyle, l'isononanoate d'isononyle, le palmitate d'éthyl 2-hexyle, le stéarate d'octyl 2-dodécyle, l'érucate d'octyl 2-dodécyle, l'isostéarate d'isostéaryle, le tridecyl trimellitate; les octanoates, décanoates ou ricinoléates d'alcools ou de polyalcools comme le dioctanoate de propylène glycol; les esters hydroxylés comme l'isostéaryl lactate, l'octyl hydroxy stéarate, l'hydroxy stéarate d'octyl dodécyle, le diisostéaryl malate, le citrate de triisocétyle, des heptanoates, octanoates, décanoates d'alcools gras; des esters de polyol comme le dioctanoate de propylène glycol, le diheptanoate de néopentyl glycol, le diisononanoate de diéthylène glycol; et les esters du pentaérythritol comme le tétra-isostéarate de pentaérythrityle;
- des alcools gras liquides à température ambiante à chaîne carbonée ramifiée et/ou insaturée ayant de 12 à 26 atomes de carbone comme l'octyldodécanol, l'alcool isostéarylique, le 2-butyloctanol, le 2-hexyldécanol, le 2-undécylpentadécanol, l'alcool oléique ;
- les acides gras supérieurs tels que l'acide oléique, l'acide linoléique, l'acide linolénique;
- les carbonates comme le diéthylhexylcarbonate;
- les acétates;
- les citrates;
- les huiles fluorées éventuellement partiellement hydrocarbonées et/ou siliconées comme les huiles fluorosiliconées, les polyéthers fluorés, les silicones fluorées telles que décrit dans le document EP-A-847752;
- les huiles siliconées comme les polydiméthylsiloxanes (PDMS) non volatiles, linéaires ou cycliques; les polydiméthylsiloxanes comportant des groupements alkyle, alcoxy ou phényle, pendant ou en bout de chaîne siliconée, groupements ayant de 2 à 24 atomes de carbone; les silicones phénylées comme les phényl triméthicones, les phényl diméthicones, les phényl triméthylsiloxy diphényl siloxanes, les diphényl diméthicones, les diphényl méthyldiphényl trisiloxanes, les 2-phényl éthyl triméthyl-siloxysilicates, et
- leurs mélanges.

La composition selon l'invention peut comprendre en outre d'autres corps gras que les huiles ci-dessus, qui peuvent être choisis par l'homme du métier sur base de ses connaissances générales, de manière à conférer à la composition finale les propriétés souhaitées, par exemple en consistance et/ou en texture.

Ces corps gras additionnels peuvent être des cires, des gommes et/ou des corps gras pâteux d'origine animale, végétale, minérale ou synthétique, ainsi que leurs mélanges.

Une cire, au sens de la présente invention, est un composé gras lipophile, solide à température ambiante (25°C), à changement d'état solide/liquide réversible, ayant une température de fusion supérieure à 40°C et pouvant aller jusqu'à 200°C, généralement une dureté supérieure à 0,5 MPa, et présentant à l'état solide une organisation cristalline anisotrope.

On peut notamment citer les cires d'origine animale, végétale, minérale ou synthétique telles que les cires microcristallines, la cire de paraffine, le pétrolatum, la vaseline, l'ozokérite, la cire de montan ; la cire d'abeilles, la cire de lanoline et ses dérivés; les cires de Candellila, d'Ouricury, de Carnauba, du Japon, le beurre de cacao, les cires de fibres de lièges ou de canne à sucre, la cire de lignite, la cire de son de riz, la cire de sapin, la cire de coton; les huiles hydrogénées ayant une température de fusion supérieure à 40°C (environ), comme l'huile de jojoba hydrogénée;
les esters gras et les glycérides concrets à 25°C; les cires de polyéthylène et les cires obtenues par synthèse de Fischer-Tropsch; des huiles hydrogénées concrètes à 25°C; des lanolines.

Les corps gras pâteux ont généralement un point de fusion compris entre 25 et 60°C, de préférence entre 30 et 45°C, et/ou une dureté allant de 0,001 et 0,5MPa, de préférence entre 0,005 et 0,4MPa. On peut notamment citer les lanolines et leurs dérivés, ou les esters de cholestérol.

### AGENTS BENEFIQUES

La quantité d'agent bénéfique présent dans les particules conformes à l'invention varie de préférence de 0,1 à 80% en poids du poids de la particule, de préférence 1 à 70% en poids, mieux de 10 à 60% et encore mieux de 15 à 50% en poids du poids total de la particule.

Le temps de libération de l'agent bénéfique variera évidemment selon la nature et l'intensité du stimulus.

La durée totale pour libérer l'agent bénéfique pourra être modulée et sera fortement dépendante de la composition de l'huile de la teneur en particules présentes dans dans l'huile de la nature notamment chimique de l'agent bénéfique et de sa concentration dans les particules (quantité encapsulée dans la particule) et de la nature et de l'intensité du stimulus auquel sera soumise la particule contenant l'agent bénéfique. La libération peut aussi bien être quasi instantanée ou durer plusieurs heures voire plusieurs jours.

Parmi les agents bénéfiques utilisables selon l'invention, on peut citer plus particulièrement
(i) les corps gras;
(ii) les substances parfumantes;
(iii) les principes actifs pharmaceutiques;
(iv) les actifs cosmétiques.

### Corps gras

Ils peuvent être choisis dans le groupe comprenant
(i) les huiles naturelles d'origine végétale, animale ou marine
(ii) les huiles minérales,
(iii) les huiles hydrogénées,
(iv) les huiles de silicone,
(v) les terpènes,
(vi) le squalène,
(vii) les acides gras saturés ou insaturés,
(viii), les esters d'acide gras,
(x) les cires,
(x) les alcools gras,
(xi) les beurres comme le beurre de karité ou le beurre de cacao,
(xii) et leurs mélanges.

### Les substances parfumantes

Par «substance parfumante», on entend tout ingrédient susceptible de dégager une odeur agréable.

Les parfums sont des compositions contenant notamment des matières premières (dénommées généralement ingrédients de parfumerie) qui sont décrites dans S. Arctander, Perfume and Flavor Chemicals (Montclair, N.J., 1969), dans S. Arctander, Perfume and Flavor Materials of Natural Origin (Elizabeth, N.J., 1960) et dans "Flavor and Fragrance Materials - 1991", Allured Publishing Co. Wheaton, III.

Il peut s'agir de produits de synthèse ou de produits naturels, comme des huiles essentielles, des absolus, des résinoïdes, des résines, des concrètes, et/ou des produits synthétiques (hydrocarbures terpéniques ou sesquiterpéniques, alcools, phénols, aldéhydes, cétones, éthers, acides, esters, nitriles, peroxydes, saturés ou insaturés, aliphatiques ou cycliques).

Selon la définition donnée dans la norme internationale ISO 9235 et adoptée par la Commission de la Pharmacopée Européenne, une huile essentielle est un produit odorant généralement de composition complexe, obtenu à partir d'une matière première végétale botaniquement définie, soit par entraînement à la vapeur d'eau, soit par distillation sèche, soit par un procédé mécanique approprié sans chauffage. L'huile essentielle est le plus souvent séparée de la phase aqueuse par un procédé physique n'entraînant pas de changement significatif de la composition.

Parmi les huiles essentielles utilisables selon l'invention, on peut citer celles obtenues à partir des plantes appartenant aux familles botaniques suivantes:
Abiétaceés ou Pinacées: conifères; Amaryllidacées; Anacardiacées; Anonacées: ylang; Apiacées (par exemple les ombellifères): aneth, angélique, coriandre, criste marine, carotte, persil; Aracées; Aristolochiacées; Astéracées: achilée, armoise, camomille, hélichryse; Bétulacées; Brassicacées; Burséracées: encen ; Caryophyllacées; Canellacées; Césalpiniacées: copaïfera (copahu); Chénopodacées; Cistacées: ciste; Cypéracées; Diptérocarpacées; Ericacées: gaulthérie (wintergreen); Euphorbiacées; Fabacées; Geraniacées: géranium; Guttifères; Hamamélidacées; Hernandiacées; Hypéricacées: millepertuis; Iridacées; Juglandacées; Lamiacées: thym, origan, monarde, sarriette, basilic, marjolaines, menthes, patchouli, lavandes, sauges, cataire, romarin, hysope, mélisse; Lauracées: ravensara, laurier, bois de rose, cannelle, litséa; Liliacées: ail, lys, muguet, jacinthe, jonquille,...; Magnoliacées: magnolia; Malvacées ; Méliacées; Monimiacées; Moracées: chanvre, houblon; Myricacées; Mysristicacées: muscade; Myrtacées: eucalyptus, tea tree, niaouli, cajeput, backousia, girofle, myrte; Oléacées; Pipéracées: poivre ; Pittosporacées; Poacées: citronnelle, lemongrass, vétiver; Polygonacées; Renonculacées; Rosacées: roses; Rubiacées; Rutacées: tous les citrus; Salicacées; Santalacées: santal; Saxifragacées; Schisandracées; Styracacées: benjoin; Thymélacées: bois d'agar; Tilliacées; Valérianacées: valériane, nard; Verbénacées: lantana, verveine; Violacées; Zingibéracées: galanga, curcuma, cardamome, gingembre; Zygophyllacées.

On peut citer également les huiles essentielles extraites de fleurs (lys, lavande, rose, jasmin, Ylang-Ylang, néroli), de tiges et de feuilles (patchouli, géranium, petit-grain), de fruits (framboise, pêche , coriandre, anis, cumin, genièvre), d'écorces de fruits (bergamote, citron, orange, pamplemousse), de racines (angélique, céleri, cardamome, iris, acore, gingembre), de bois (bois de pin, santal, gaïac, cèdre rose, camphre), d'herbes et de graminées (estragon, romarin, basilic, lemon grass, sauge, thym), d'aiguilles et de branches (épicéa, sapin, pin, pin nain), de résines et de baumes (galbanum, élémi, benjoin, myrrhe, oliban, opopanax).

Des exemples de substances parfumantes sont notamment : le géraniol, l'acétate de géranyle, le farnésol, le bornéol, l'acétate de bornyle, le linalool, l'acétate de linalyle, le propionate de linalyle, le butyrate de linalyle, le tétrahydrolinalool, le citronellol, l'acétate de citronellyle, le formiate de citronellyle, le propionate de citronellyle, le dihydromyrcénol, l'acétate de dihydromyrcényle, le tétrahydromyrcénol, le terpinéol, l'acétate de terpinyle, le nopol, l'acétate de nopyle, le nérol, l'acétate de néryle, le 2-phényléthanol, l'acétate de 2-phényléthyle, l'alcool benzylique, l'acétate de benzyle, le salicylate de benzyle, l'acétate de styrallyle, le benzoate de benzyle, le salicylate d'amyle, le diméthylbenzyl-carbinol, l'acétate de trichlorométhylphénylcarbinyle, l'acétate de p-tert-butylcyclohexyle, 'acétate d'isononyle, l'acétate de cis-3-hexenyle, l'acétate de vétivéryle, l'acétate d'éthyle, l'acétate de butyle, l'acétate d'hexyle, l'acétate de décyle, l'acétate d'isoamyle, l'acétate de stéaryle, l'heptanoate d'allyle, le vétivérol, l'alpha-hexylcinnamaldéhyde, le 2-méthyl-3-(p-tert-butylphényl)propanal, le 2-méthyl-3-(p-isopropylphényl)propanal, le 3-(p-tert-butylphényl)-propanal, le 2,4-diméthylcyclohex-3-enyl-carboxaldéhyde, l'acétate de tricyclodécènyle, le propionate de tricyclodécènyle, l'allyl 3-cyclohexylpropionate, l' éthyl-6-(acétyloxy)-hexanoate, le caproate d'allyle, l' éthyl-2 méthyl butyrate, le méthyl dihydrojasmonate, le salicylate d'hexyle, le 4-(4-hydroxy-4-méthylpentyl)-3-cyclohexènecarboxaldéhyde, le 4-(4-méthyl-3-pentènyl)-3-cyclohexènecarboxaldéhyde, le 4-acétoxy-3-pentyl-tétrahydropyrane, le 3-carboxyméthyl-2-pentylcyclopentane, la 2-n-4-heptylcyclopentanone, la 3-méthyl-2-pentyl-2-cyclopentènone, la menthone, la carvone, la tagétone, la géranyl acétone, le n-décanal, le n-dodécanal, l' anisyl propanal, le 9-décènol-1, le cis-3-hexénol, le tétrahydro-2-isobutyl-4-méthylpyrann-4-ol, 3-Methyl-5-phényl-1-pentanol, le 3a,6,6,9a-Tétraméthyl-dodécahydronaphtho[2,1-b]furanne, l'isobutyrate de phénoxyéthyle, le phényl-acétaldéhyde diméthyl-acétal, le phénylacétaldéhyde diéthylacétal, le géranonitrile, le citronellonitrile, l'acétate de cédryle, le 3-isocamphylcyclohexanol, le cédryl méthyl éther, l'isolongifolanone, l'aubépinonitrile, l'aubépine, l'héliotropine, la coumarine, l'eugénol, la vanilline, l'oxyde de diphényle, le citral, le citronellal, l'hydroxycitronellal, l'hexylcinnamal, le 2,4-dimethylcyclohex-3-ène-1-carbaldéhyde, le 2,6-dimethylhept-5-ènal, l' α,α-diméthyl-p-éthylphénylpropanal, le 1,3-benzodioxole-5-carboxaldéhyde, le limonène, la damascone, la décalactone, la nonalactone, le 6,6-diméthoxy-2,5,5-triméthylhex-2-ène, la 2,4,4,7-tétraméthyloct-6-èn-3-one, la 1-(5,5-diméthyl-1-cyclohexenyl)-pent-4-èn-1-one, la méthylheptènone, le 4-(cyclopropylméthyl)-phénylméthyl éther, le 2-methyl-6-methylideneoct-7-en-2-ol, l'oxyde de rose, la 1-(1,2,3,4,5,6,7,8-octahydro-2,3,8,8-tetraméethyl-2-naphthyl)éthan-1-one, la 2-acétonaphthone, la 2-isopropyl-5-méthylcyclohexanone, les ionones, les méthylionones, les isométhylionones, la solanone, les irones, le cis-3-hexénol et ses esters, les muscs-indanes, les muscs-tétralines, les muscs-isochromanes, les cétones macrocycliques, les muscs-macrolactones, les muscs aliphatiques, le brassylate d'éthylène, l'essence de rose et leurs mélanges.

D'une façon générale, les parfums sont constitués d'un mélange d'ingrédients dits de parfumerie qui peuvent être également classés en notes de tête, notes de cœur et notes de fond.

Les trois notes correspondent à la volatilité plus ou moins importante des ingrédients qui les composent: note de tête fortement volatile, note de coeur moyennement volatile et note de fond faiblement volatile.
(i) La note de tête appelée aussi «départ» est celle que l'odorat perçoit en premier dès que le parfum est en contact avec la matière kératinique ou tout substrat. Mais c'est celle qui s'atténue le plus rapidement: elle ne "tient pas". Il est difficile d'exprimer le temps de persistance de cette note, car il est très variable: de quelques minutes à une dizaine de minutes
   Elle est essentiellement fraîche et légère. Tous les agrumes appartiennent notamment à cette catégorie. En parfumerie, on les range sous le terme générique d'hespéridés dont font partie orange, citron, pamplemousse, bergamote, néroli etc... On citera également les aromates tels que la lavande, le laurier, le thym ou le romarin et les anisés, mentholés, aldéhydés, etc. On citera également les notes eucalyptus.
(ii) La note de cœur, parfois appelée aussi «corps» a une persistance qui va de quelques dizaines de minutes à quelques heures, mais sa principale caractéristique est de ne se révéler qu'au bout de quelques minutes. Elle "démarre" donc juste avant l'extinction de la note de tête. Elle commence à s'exprimer alors que la note de tête s'efface progressivement. Elle est représentée essentiellement par des éléments floraux, fruités ou épicés: muguet, chèvrefeuille, violette, magnolia, cannelle, géranium, jasmin, rose, iris, framboise, pêche, etc...
(ii) La note de fond, parfois appelée aussi «fond» assure la "durabilité", la persistance ou la ténacité d'un parfum. Elle est perceptible plusieurs heures, voire plusieurs jours, ou même plusieurs semaines après application. sur un vêtement ou sur une mouillette ou touche olfactive, selon la concentration du parfum. On citera par exemple les bois, racines, mousses, résines et les substances animales ou minérales telles que opoponax, muscs, ambre, santal, benjoin, lichen, clou de girofle, sauge, etc. On citera également les notes vanillées, le patchouli, les coumarines...etc.

On peut bien entendu encapsuler des ingrédients appartenant à une ou plusieurs notes. Toutefois, on préférera encapsuler les ingrédients les plus volatiles (= les moins rémanents) appartenant aux notes de tête et/ou de cœur. Parmi ces ingrédients, on citera, par exemple :
Acétate de benzyle
Acétate de géranyle
Acétate de cis-3-hexenyle
Aldéhyde C18 ou nonalactone
Acétate de décyle
Allyl amyl glycolate (citral)
Acétate d'éthyle
Acétate de butyle
Allyl 3-cyclohexylpropionate
Acétate de linalyle
Alcool Phényléthylique
Acétate d'héxyle
Berryflor ou éthyl-6-(acétyloxy)-hexanoate
Acétate d'isoamyle
Caproate d'allyle
Amarocite ou 6,6-diméthoxy-2,5,5-triméthylhex-2-ène
Citral lemarome N ou 3,7-diméthylocta-2,6-diénal
Canthoxal ou anisyl propanal
Claritone ou 2,4,4,7-tétraméthyloct-6-èn-3-one
Ethyl-2méthyl butyrate
Dihydromyrcénol
Cis-3 hexenol
Hédione ou méthyl dihydrojasmonate
L-carvone
Heptanoate d'allyle
Limonène
Néobuténone alpha ou 1-(5,5-diméthyl-1-cyclohexenyl)- pent-4-èn-1-one
Méthylheptènone
Toscanol ou 4-(cyclopropylméthyl)-phénylméthyl éther
myrcenol super ou 2-methyl-6-methylideneoct-7-en-2-ol
Decalactone
Acétate de stéaryle
Oxyde de rose
Linalool
Triplai ou 2,4-dimethylcyclohex-3-ène-1-carbaldéhyde
Mélonal ou 2,6-dimethylhept-5-ènal
1-(1,2,3,4,5,6,7,8-octahydro-2,3,8,8-tetraméethyl-2-naphthyl)éthan-1-one
Hexylcinnamal
Tétrahydro-2-isobutyl-4-méthylpyrann-4-ol
Salicylate d'hexyle
1,4-Dioxacycloheptadécane-5,17-dione
et leurs mélanges

Selon une forme particulière de l'invention, les particules d'encapsulation comprennent au moins une ou plusieurs substances parfumantes ayant une pression de vapeur saturante à 25°C supérieure ou égale à 10,0Pa.

La pression de vapeur saturante (ou tension de vapeur) est la pression à laquelle la phase gazeuse d'une substance est en équilibre avec sa phase liquide ou solide à une température donnée dans un système fermé. Le calcul de la pression de vapeur saturante peut se faire à l'aide de la formule suivante : 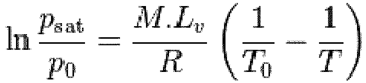 avec :
T₀ : température d'ébullition de la substance à une pression p₀ donnée, en degrés Kelvin,
psat : pression de vapeur saturante, dans la même unité que p0
M : masse molaire de la substance, en kg/mol
Lv : chaleur latente de vaporisation de la substance, en Joules/kg
R : constante des gaz parfaits, égale à 8,31447 J/K/mol
T : température de la vapeur, en K.

De préférence les substances parfumantes ayant une pression de vapeur saturante à 25°C supérieure ou égale à 10Pa représentent une quantité allant de 50 à 100% en poids, de préférence de 60 à 100% en poids, plus préférentiellement de 70 à 100% en poids, et encore mieux de 80 à 100% en poids par rapport au poids total des substances parfumantes présentes dans les particules de l'invention.

### a) Les principes actifs pharmaceutiques

On entend par « principe actif pharmaceutique » une molécule ou un mélange de molécules qui possède un effet thérapeutique, curatif et/ou prophylactique pouvant être administré par pulvérisation.

### b) Les actifs cosmétiques

On entend par «actif cosmétique» toute molécule qui possède un effet d'hygiène, de soin, de maquillage, de coloration contribuant à l'amélioration, du bien-être et/ou à l'embellissement ou la modification de l'aspect de la matière kératinique humaine sur laquelle on applique ladite composition.

Parmi les actifs cosmétiques susceptibles d'être appliqués sur des matières kératiniques humaines telles que la peau, les lèvres, le cuir chevelu, les cheveux, les cils ou les ongles, on peut citer par exemple, seuls ou en mélanges :
- les vitamines et leurs dérivés ou précurseurs, seuls ou en mélanges,
- les agents anti-oxydants;
- les agents nettoyants tels que les tensio-actifs;
- les matières colorantes;
- les agents conditionneurs,
- les agents pour le défrisage et/ou le lissage et/ou la mise en forme des cheveux ;
- les agents anti-radicalaires;
- les agents photoprotecteurs comme les filtres UV organiques ou inorganiques,
- les agents autobronzants,
- les agents anti-glycation;
- les agents apaisants,
- les agents dépilatoires,
- les agents déodorants,
- les agents anti-transpirants,
- les inhibiteurs de NO-synthase;
- les agents stimulant la prolifération des fibroblastes;
- les agents stimulant la prolifération des kératinocytes;
- les agents dermorelaxants,
- les agents rafraîchissants,
- les agents tenseurs,
- les agents matifiants,
- les agents anti-luisance de la peau,
- les agents anti-séborrhéiques,
- les agents anti-cheveux gras,
- les agents dépigmentants,
- les agents pro-pigmentants,
- les agents kératolytiques,
- les agents desquamants;
- les agents hydratants,
- les agents anti-microbiens,
- les agents amincissants,
- les agents agissant sur le métabolisme énergétique des cellules,
- les agents répulsifs contre les insectes,
- les antagonistes de substances P ou de CRGP ;
- les agents anti-chute des cheveux ;
- les agents anti-rides,
- les agents anti-vieillissement ;
- les agents antipelliculaires.

Parmi ces actifs cosmétiques, on préférera tout particulièrement, seuls ou en mélanges :
- les agents photoprotecteurs comme les filtres UV en particulier les filtres UV organiques;
- les agents anti-luisance de la peau,
- les agents dermorelaxants,
- les agents anti-séborrhéiques,
- les agents anti-cheveux gras,
- les agents déodorants,
- les agents anti-transpirants,
- les agents rafraîchissants,
- les agents matifiants,
- les agents anti-microbiens,
- les agents antipelliculaires.

Selon une forme particulièrement préférée de l'invention, le ou les agents bénéfiques présents dans les particules seront choisis parmi les substances parfumantes.

Selon une forme encore plus particulièrement préférée de l'invention, les substances parfumantes présentes dans les particules sont choisies parmi les notes de cœur et/ou les notes de tête de façon à pouvoir aussi bien compenser leur perte tout au long de la journée qu'à procurer un effet fraicheur supplémentaire au cours de la journée en réponse à la transpiration comme à l'humidité atmosphérique ou apportée par exemple par des brumisateurs.

Selon une forme particulière de l'invention, la composition contiendra
a) des particules renfermant au moins une substance parfumante et
b) au moins une substance parfumante sous forme libre, identique ou différente de la substance parfumante présente dans lesdites particules.

Lesdites substances parfumantes sous forme libre peuvent être choisies parmi celles citées précédemment.

Selon une autre forme particulière de l'invention, la composition contient exclusivement la ou les substances parfumantes dans les particules d'encapsulation. Autrement dit, la totalité des ingrédients pour parfumer présents dans la composition sont contenus dans les particules.

La composition peut comprendre, en outre, d'autres ingrédients sous forme libre (non encapsulés, emprisonnés dans les particules de l'invention) utilisés couramment dans les compositions cosmétiques. De tels ingrédients peuvent être choisis parmi les antioxydants, les conservateurs, les actifs cosmétiques tels que ceux cités précédemment, les substances parfumantes telles que celles décrites précédemment, les tensioactifs, les agents d'étalement, les agents mouillants, les agents dispersants, les anti-mousses, les neutralisants, les stabilisants, les polymères et notamment les polymères filmogènes liposolubles, et leurs mélanges.

Bien entendu, l'homme du métier veillera à choisir ce ou ces éventuels composés complémentaires, et/ou leur quantité, de manière telle que les propriétés avantageuses de la composition pour l'utilisation selon l'invention ne soient pas, ou substantiellement pas, altérées par l'adjonction envisagée.

Les compositions selon l'invention peuvent se présenter sous toute forme acceptable et usuelle pour une composition sous forme d'huile.

L'homme du métier pourra choisir la composition appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

Selon une forme particulière de l'invention, les compositions selon l'invention sont des produits de soin de la peau notamment du visage ou des lèvres où la composition comprend au moins un actif cosmétique ou dermatologique. Ces produits peuvent être notamment des huiles solaires, des huiles auto-bronzantes, des huiles de massage. Plus particulièrement, les particules comprennent au moins une substance parfumante. Encore plus particulièrement, les compositions contiendront en plus une substance parfumante sous forme libre, identique ou différente de la substance parfumante présente dans les particules.

Selon une autre forme particulière de l'invention, les compositions selon l'invention peuvent se présenter sous forme de produits capillaires. Ces produits capillaires peuvent être notamment des produits de soin, de conditionnement, de coiffage. Plus particulièrement, les particules comprennent au moins une substance parfumante. Encore plus particulièrement, les compositions contiendront en plus une substance parfumante sous forme libre, identique ou différente de la substance parfumante présente dans les particules.

Selon une autre forme particulière de l'invention, les compositions selon l'invention peuvent se présenter sous forme de produits déodorants et/ou anti-transpirants où la composition comprend au moins un actif déodorant et/ou au moins un actif anti-transpirant sous forme libre et/ou sous forme encapsulée. Plus particulièrement, les particules comprennent au moins une substance parfumante. Encore plus particulièrement, les compositions contiendront en plus une substance parfumante sous forme libre, identique ou différente de la substance parfumante présente dans les particules.

### Actif anti-transpirant

Par «actif anti-transpirant», on entend un composé qui, à lui seul, a pour effet de diminuer le flux sudoral, et/ou de diminuer la sensation sur la peau d'humidité liée à la sueur humaine et/ou d'absorber partiellement ou totalement la sueur humaine.

Parmi les actifs anti-transpirants, on peut citer les sels d'aluminium et/ou de zirconium tels que le chlorhydrate d'aluminium, l'aluminium chlorohydrex, l'aluminium chlorohydrex PEG, l'aluminium chlorohydrex PG, l'aluminium dichlorohydrate, l'aluminium dichlorohydrex PEG, l'aluminium dichlorohydrex PG, l'aluminium sesquichlorohydrate, l'aluminium sesquichlorohydrex PEG, l'aluminium sesquichlorohydrex PG, les sels d'alun, l'aluminium sulfate, l'aluminium zirconium octachlorohydrate, l'aluminium zirconium pentachlorohydrate, l'aluminium zirconium tetrachlorohydrate, l'aluminium zirconium trichlorohydrate et plus particulièrement le chlorhydrate d'aluminium sous forme activée ou non commercialisé par la société REHEIS sous la dénomination MICRODRY ALUMINUM CHLOROHYDRATE® ou par la société GUILINI CHEMIE sous la dénomination ALOXICOLL PF 40. Des sels d'aluminium et de zirconium sont par exemple celui commercialisé par la société REHEIS sous la dénomination REACH AZP-908-SUF®, des sel d'aluminium «activés» par exemple celui commercialisé par la société REHEIS sous la dénomination REACH 103 ou par la société WESTWOOD sous la dénomination WESTCHLOR 200.

De préférence, la composition cosmétique comprend le chlorhydrate d'aluminium en tant qu'actif anti-transpirant.

Comme autre actif anti-transpirant, on peut citer les particules de perlite expansée telles que celles obtenues par le procédé d'expansion décrit dans le brevet US 5,002,698.

Les perlites utilisables selon l'invention sont généralement des aluminosilicates d'origine volcanique et ont comme composition
70,0-75,0% en poids de silice SiO₂
12,0-15,0% en poids d'oxyde d'aluminium oxyde Al₂O₃
3,0-5,0% d'oxyde de sodium Na₂O
3,0-5,0% d'oxyde de potassium K₂O
0,5-2% d'oxyde de fer Fe₂O₃
0,2-0,7% d'oxyde de magnesium MgO
0,5-1,5% d'oxyde de calcium CaO
0,05 - 0,15% d'oxyde de titane TiO₂

De préférence, les particules de perlite utilisées seront broyées ; elles sont dans ce cas dites Expanded Milled Perlite (EMP). Elles ont de préférence une taille de particule définie par un diamètre médian D50 allant de 0,5 à 50µm et de préférence de 0,5 à 40µm.

De préférence, les particules de perlite utilisées présentent une densité apparente non tassée à 25°C allant de 10 et 400kg/m3 (Norme DIN 53468) et de préférence de 10 et 300kg/m3.

De préférence, les particules de perlite expansée selon l'invention ont une capacité d'absorption d'eau mesurée au WET POINT allant de 200 à 1500% et de préférence de 250 à 800%.

Le Wet Point correspond à la quantité d'eau qu'il faut additionner à 100g de particule pour obtenir une pâte homogène. Cette méthode dérive directement de celle de la prise d'huile appliquée aux solvants. Les mesures sont faites de la même manière par l'intermédiaire du Wet Point et du Flow Point ayant respectivement comme définition suivante:
WET POINT : masse exprimée en grammes pour 100g de produit correspondant à l'obtention d'une pâte homogène lors de l'addition d'un solvant à une poudre.

FLOW POINT: masse exprimée en grammes pour 100g de produit à partir de laquelle la quantité de solvant est supérieure à la capacité de la poudre à le retenir. Cela se traduit par l'obtention d'un mélange plus ou moins homogène s'écoulant sur la plaque de verre.

Le Wet Point et le Flow point sont mesurés selon le protocole suivant:

### Protocole de mesure de l'absorption d'eau.

### 1) Matériel utilisé

Plaque de verre (25 x 25 mm)
Spatule (manche en bois et partie métallique (15 x 2,7mm)
Pinceau à poils de soie
Balance

### 2) Mode Opératoire

On dépose la plaque de verre sur la balance et on pèse 1g de particules de perlite. On dépose le bécher contenant le solvant ainsi que la liquipipette de prélèvement sur la balance. On ajoute progressivement le solvant à la poudre en malaxant régulièrement l'ensemble (toutes les 3 à 4 gouttes) à l'aide de la spatule

On note la masse de solvant nécessaire à l'obtention du Wet Point. On ajoute à nouveau le solvant et on note la masse permettant d'arriver au Flow Point. On effectuera la moyenne sur 3 essais.

On utilisera en particulier les particules de perlite expansée vendues sous les noms commerciaux OPTIMAT 1430 OR ou OPTIMAT 2550 par la société WORLD MINERALS.

### Actifs déodorants

On appelle « actif déodorant », toute substance capable de masquer, absorber, améliorer et/ou réduire l'odeur désagréable résultant de la décomposition de la sueur humaine par des bactéries

Les actifs déodorants peuvent être des agents bactériostatiques ou des agents bactéricides agissant sur les germes des odeurs axillaires, comme le 2,4,4'-trichloro-2'-hydroxydiphényléther (®Triclosan), le 2,4-dichloro-2'-hydroxydiphényléther, le 3',4',5'-trichlorosalicylanilide, la 1-(3',4'-dichlorophenyl)-3-(4'-chlorophenyl)urée (®Triclocarban) ou le 3,7,11-triméthyldodéca-2,5,10-triénol (®Farnesol) ; les sels d'ammonium quaternaires comme les sels de cetyltrimethylammonium, les sels de cétylpyridinium ; les polyols comme ceux de type glycérine, 1,3-propanediol (ZEMEA PROPANEDIOL® commercialisé par Dupont Tate and Lyle Bioproducts), le 1,2-décanediol (SYMCLARIOL® de la société Symrise) ; les dérivés de glycérine comme par exemple le Caprylic/Capric Glycerides (CAPMUL MCM® de Abitec), le Caprylate ou caprate de Glycerol (DERMOSOFT GMCY® et DERMOSOFT GMC® respectivement de STRAETMANS), le Polyglyceryl-2 Caprate (DERMOSOFT DGMC® de STRAETMANS) les dérivés de biguanide comme les sels de polyhexaméthylène biguanide ; la chlorhexidine et ses sels; le 4-Phenyl-4,4-dimethyl-2butanol (SYMDEO MPP® de Symrise) ; les cyclodextrines ; les chélatants tels que le Tetrasodium Glutamate Diacetate (CAS #51981-21-6) vendu sous le nom commercial DISSOLVINE GL-47-S® de Akzo Nobel, l'EDTA (acide Ethylendiamino Tétraacétique) et le DPTA (acide 1,3-diaminopropanetétraacétique).

Parmi les actifs déodorants conformes à l'invention, on peut aussi citer également
- les sels de zinc comme le salicylate de zinc, le phénolsulfonate de zinc, le pyrrolidone carboxylate de zinc (plus communément appelé pidolate de zinc), le sulfate de zinc, le chlorure de zinc, le lactate de zinc, le gluconate de zinc, ricinoléate de zinc, glycinate de zinc, carbonate de zinc, citrate de zinc, chlorure de zinc, le laurate de zinc, l'oléate de zinc, l'orthophosphate de zinc, le stéréate de zinc, le tartrate de zinc, l'acétate de zinc ou leurs mélanges ;
- des absorbeurs d'odeurs comme les zéolites notamment métalliques sans argent, les cyclodextrines, les silicates d'oxyde métallique telles que celles décrite dans la demande US2005/063928 ; des particules d'oxyde métallique modifiées par un métal de transition telles que décrites dans les demandes US2005084464 et US2005084474, des aluminosilicates comme ceux décrits dans la demande EP1658863, des particules de dérivés de chitosan comme celles décrites dans le brevet US6916465 ;
- le bicarbonate de sodium ;
- l'acide salicylique et ses dérivés tels que l'acide n-octanoyl-5-salicylique
- l'alun.
- le triéthyl citrate

Les actifs déodorants peuvent être présents de préférence dans les compositions selon l'invention dans des concentrations pondérales allant 0,01 à 10% en poids par rapport au poids total de la composition.

La présente invention concerne également un procédé cosmétique de traitement des odeurs corporelles et éventuellement de la transpiration humaine consistant à appliquer sur la matière kératinique une composition comprenant les particules telles que définies précédemment; ladite composition comprenant au moins un actif déodorant et/ou au moins un actif anti-transpirant, sous forme libre et/ou sous forme encapsulée.

L'invention est illustrée plus en détail dans les exemples suivants.

### Exemples de préparation de particules à libération de parfum

### Exemple A

On a préparé des capsules en mettant en œuvre la composition suivante:

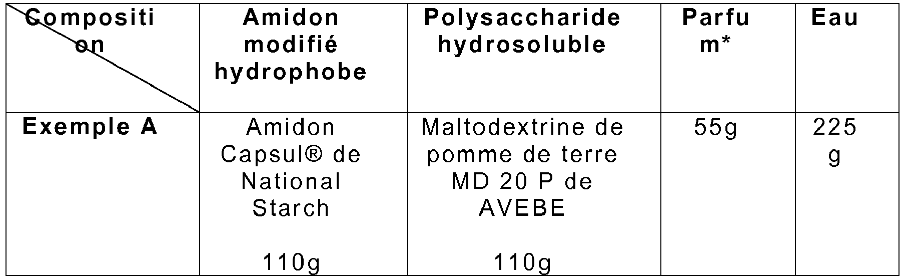

* Le parfum utilisé a la composition suivante :

| **Ingrédients** | **Quantité en g** |
|---|---|
| Myristate d'isopropyle | 20,5 |
| Méthyl dihydrojasmonate | 15 |
| 2-phenyléthanol | 8 |
| 1-(1,2,3,4,5,6,7,8-octahydro-2,3,8,8-tetraméthyl-2-naphthyl)éthan-1-one | 8 |
| Hexylcinnamal | 6 |
| Tétrahydro-2-isobutyl-4-méthylpyrann-4-ol | 6 |
| Hexyl Salicylate | 6 |
| Benzyl Acétate | 5 |
| 1,4-Dioxacycloheptadécane-5,17-dione | 5 |
| 3-Methyl-5-phényl-1-pentanol | 5 |
| dihydromyrcenol | 4 |
| ORANGE TERPENES 0,05% B H T | 4 |
| (limonène >95%) | |
| 2-acétonaphthone | 2 |
| 3a,6,6,9a-Tétraméthyl-dodécahydronaphtho[2,1-b]furanne | 1 |
| α,α-diméthyl-p-éthylphénylpropanal | 1 |
| 1,3-benzodioxole-5-carboxaldéhyde | 1 |
| 2-isopropyl-5-méthylcyclohexanone | 1 |
| 1-phényléthyl acetate | 0,8 |
| 2,6-dimethylhept-5-ènal (mélonal) | 0,5 |
| 2,4-dimethylcyclohex-3-ène-1-carbaldehyde (triplai) | 0,2 |

### Procédé de préparation de l'émulsion

On a mélangé la maltodextrine de pomme de terre MD20 P et l'amidon CAPSUL® (sel de sodium de l'octényle succinate d'amidon) dans l'eau jusqu'à dissolution puis on a ajouté le parfum et émulsionné avec un disperseur Ultraturrax de type Heidolph Diax 900 (moteur de puissance 900W avec une vitesse de 8000 à 26000 tour/mn électroniquement contrôlée) à la puissance maximale pendant 4 minutes

### Procédure de séchage pour obtenir des particules sphériques

L'émulsion obtenue a été ensuite homogénéisée à une pression de 30 bars au moyen d'une pompe à haute pression puis pulvérisée dans une chambre d'atomisation au moyen d'une buse simultanément avec un courant de CO₂ (30 bars, 45°C) que l'on a fait circuler en continu avec un débit d'environ 500g/mn pour éliminer l'eau. La poudre séchée a été retenue sur un filtre situé à la base de la chambre d'atomisation puis collectée après dépressurisation. On obtient ainsi 270g de microcapsules sphériques sous la forme d'une fine poudre blanche ayant un diamètre moyen en nombre de 7,8 µm et un diamètre moyen en volume de 47 µm.

La taille des particules a été mesurée en voie sèche par diffraction laser en utilisant un granulomètre Microtrac S3500, les granulométries étant exprimées en volume et en nombre.

| **Exemple A** | **Caractéristiques mesurées des capsules** | | | |
|---|---|---|---|---|
| | **Taux de parfum encapsulé (%)** | **Taux de parfum libre (%)** | **Densité de poudre versée** | **Densité absolue** |
| | 19,8 | < 0,1 | 484 | 1,12 |

### Exemples B à H (Exemple H est hors invention)

Selon le procédé décrit à l'exemple A, on a préparé les capsules suivantes :

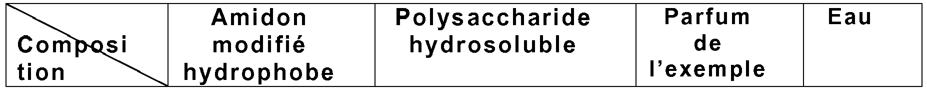

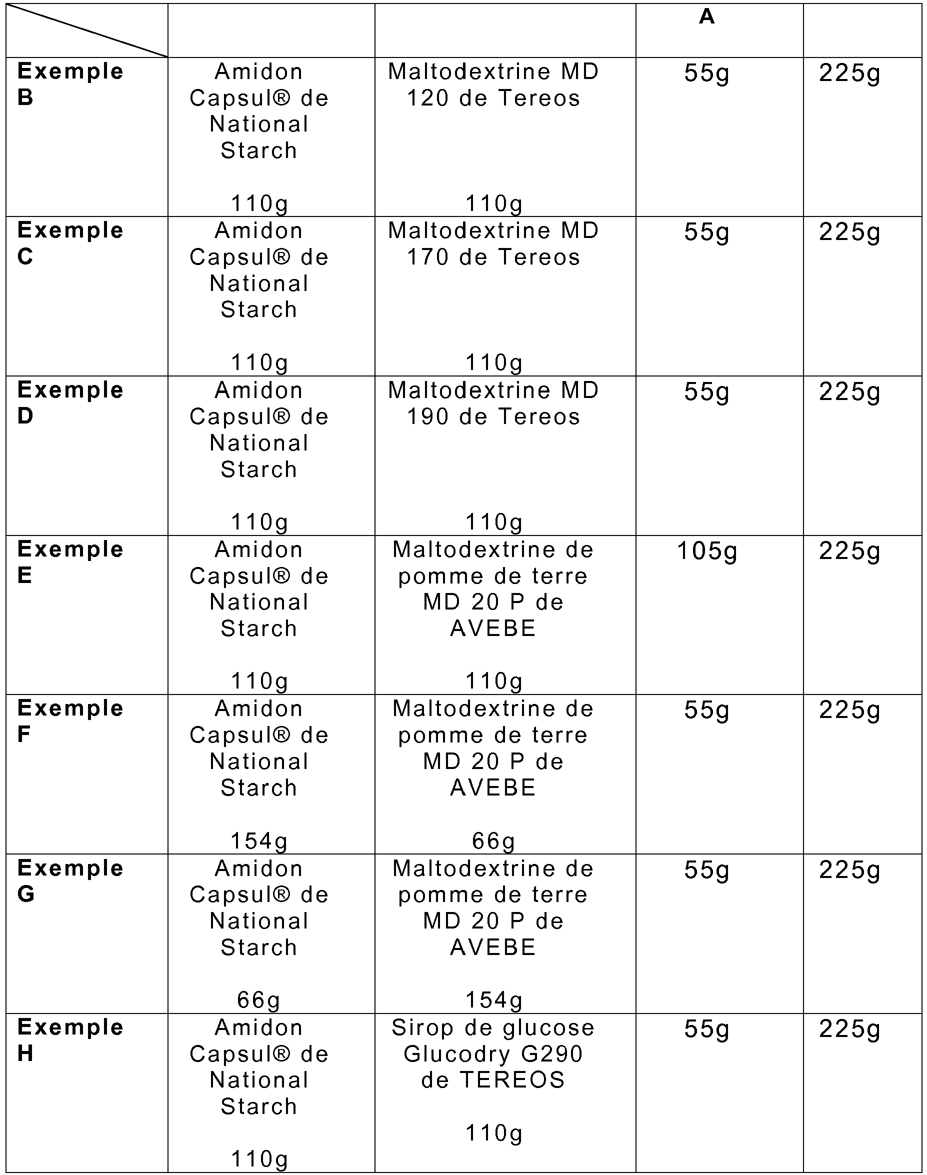

| **Exemples** | **Caractéristiques mesurées des capsules** | | | |
|---|---|---|---|---|
| | **Taux de parfum encapsulé (%)** | **Taux de parfum libre (%)** | **Densité de poudre versée (g/l)** | **Densité absolue** |
| **Exemple B** | 19,3 | <0,1 | 568 | 1,14 |
| **Exemple C** | 19,4 | <0,1 | 490 | 1,16 |
| **Exemple D** | 19,9 | <0,1 | 537 | 1,11 |
| **Exemple E** | 38 | 0,8 | 482 | 1,08 |
| **Exemple F** | 21,0 | 0,2 | 595 | 1,11 |
| **Exemple G** | 20,7 | 0,2 | 521 | 1,15 |
| **Exemple H** | 19,2 | 0,1 | 568 | 1,12 |

### Exemple I comparatif

On a réalisé des capsules ayant la même composition que l'exemple A tel décrit ci-dessus selon le procédé de l'exemple 1 du brevet US6200949 comprenant un séchage par spray-drying (atomisation) de l'émulsion.

L'émulsion est séchée par spray-drying au moyen d'un appareil du type Bowen Lab Model Dryer utilisant de l'air avec un débit de 420m³/h à une température de 204°C et une température externe de 93°C et une vitesse de la turbine de 50000 tr/mn.

Aspect morphologique des particules obtenues: polymorphe avec agrégats

### Exemple J comparatif

On a réalisé des capsules ayant la même composition que l'exemple A tel décrit ci-dessus selon le procédé de l'exemple 1 du du brevet US5508259 comprenant un séchage par spray-drying (atomisation) de l'émulsion..

Le mélange a été séché par spray-drying avec un appareil du type CCM Sulzer avec un débit d'émulsionde 50kg/h, de l'air à un débit de 320m³/h à 350°C et 0,45 bar.

Aspect morphologique des particules obtenues : polymorphe avec agrégats

| **Composition** | **Caractéristiques mesurées des capsules** | | | |
|---|---|---|---|---|
| | **Taux de parfum encapsulé (%)** | **Taux de parfum libre (%)** | **Densité de poudre versée (g/l)** | **Densité absolue** |
| **Exemple I (hors invention)** | 18,3 | 2,7 | 259 | 1,16 |
| **Exemple J (hors invention)** | 11,2 | 1,7 | 269 | 1,12 |

### Exemple 1: Huile solaire

On a préparé une huile solaire ayant la composition suivante :

| **Ingrédients** | **(% en poids)** |
|---|---|
| Cyclopentadimethylsiloxane | 20 |
| Huile de ricin | 1,8 |
| 4-hydroxycinnamate de 2-éthylhexyle | 0,1 |
| Capsules de parfum de l'exemple A | 0,6 |
| Palmitate isopropyle | qs |
| Total | 100,00 |

On introduit dans une cuve équipée d'un agitateur 10,0g de cyclopenta dimethylsiloxane, 0,9g d' huile de ricin, 0,05g d'hydroxycinnamate d'ethylhexyle et 38,75g de palmitate d'isopropyle. On homogénéise le mélange sous agitation vive (1000tr/mn). Puis on ajoute 0,3g de capsules de l'exemple A. On homogénéise à nouveau le mélange sous agitation vive (1000 tr/mn). On a ainsi obtenu un liquide blanc-jaunâtre

### Exemples C1 et C2 comparatifs: Huiles solaires

Similairement à l'exemple1, on a préparé des huiles solaires anhydres ayant les compositions suivantes :

**Exemple C1**

| **Ingrédients** | **(% en poids)** |
|---|---|
| Cyclopentadimethylsiloxane | 20 |
| Huile de ricin | 1,8 |
| 4-hydroxycinnamate de 2-éthylhexyle | 0,1 |
| Capsules de parfum de l'exemple I | 0,6 |
| Palmitate isopropyle | qs |
| Total | 100,00 |

**Exemple C2**

| **Ingrédients** | **(% en poids)** |
|---|---|
| Cyclopentadimethylsiloxane | 20 |
| Huile de ricin | 1,8 |
| 4-hydroxycinnamate de 2-éthylhexyle | 0,1 |
| Capsules de parfum de l'exemple J | 0,6 |
| Palmitate isopropyle | qs |
| Total | 100,00 |

### Protocole d'évaluation

On a déposé de façon homogène environ 0,2 g de composition sur une mouillette. Après une minute on a évalué intensité odeur de parfum. Puis on a simulé la transpiration par un ajout d'eau d'environ 0,1g (trois sprays) sur la composition déposée. On a attendu une minute et on a à nouveau senti.

| **Huile** | **Intensité odeur AV** | **Intensité odeur AP** |
|---|---|---|
| | | |
| Exemple 1 | inodore | Forte odeur de parfum |
| Exemple C1 | Forte odeur de parfum | Forte odeur de parfum |
| Exemple C2 | Forte odeur de parfum | Forte odeur de parfum |

| | | |
|---|---|---|
| AV = avant ajout d'eau ; AP = après ajout d'eau ; | | |

On a ainsi observé à T₀ que l'huile de l'exemple 1 comprenant les capsules de parfum selon l'invention ne présente aucune odeur avant l'ajout d'eau contrairement aux huiles C1 et C2 (hors invention), ce qui montre que les capsules de parfum dans les huiles C1 et C2 ne sont pas étanches avant même l'ajout d'eau.

On a également observé que l'huile de l'exemple 1 après stimulation à l'eau, conduisait à une odeur très intense, ce qui montre une libération importante de parfum en réponse au stimuli eau

Similairement à l'exemple1, on a préparé des huiles solaires anhydres ayant les compositions suivantes:

### Exemple 2 : Huile solaire

| **Ingrédients** | **(% en poids)** |
|---|---|
| Cyclopentadimethylsiloxane | 20 |
| Huile de ricin | 1,8 |
| 4-hydroxycinnamate de 2-éthylhexyle | 0,1 |
| Capsules de parfum de l'exemple B | 0,6 |
| Palmitate isopropyle | qs |
| Total | 100,00 |

Les capsules de parfum de l'exemple B peuvent être remplacées par les capsules des exemples A et C à H décrites précédemment

On a ajouté dans une cuve équipée d'un agitateur 10,0g de cyclopenta dimethylsiloxane, 0,9g d'huile de ricin, 0,05g d'hydroxycinnamate d'éthylhexyle et 38,75g de palmitate d'isopropyle. On a homogénéisé le mélange sous agitation vive (1000tr/mn). Puis on a ajouté 0,3g de capsules de l'exemple B. On a homogénéisé à nouveau le mélange sous agitation vive (1000 tr/mn). On a ainsi obtenu un liquide blanc-jaunâtre.

La composition appliquée sur le corps libère le parfum au cours de la journée au contact de la transpiration ou de l'humidité.

### Exemple 3 : Huile solaire

| **Ingrédients** | **(% en poids)** |
|---|---|
| Cyclopentadimethylsiloxane | 20 |
| Huile minérale | 1,8 |
| 4-hydroxycinnamate de 2-éthylhexyle | 0,1 |
| Parfum A | 0,2 |
| Capsules de parfum de l'exemple F | 0,6 |
| Palmitate isopropyle | qs |
| Total | 100,00 |

Les capsules de parfum de l'exemple F peuvent être remplacées par les capsules des exemples A à E et G, H décrites précédemment

On a ajouté dans une cuve équipée d'un agitateur 10,0g de cyclopenta dimethylsiloxane, 0,9g d' huile minérale, 0,05g d'hydroxycinnamate d'éthylhexyle, 0,1g de Parfum A et 38,65g de palmitate d'isopropyle. On a homogénéisé le mélange sous agitation vive (1000tr/mn). Puis on ajoute 0,3g de capsules de l'exemple F. On homogénéise à nouveau le mélange sous agitation vive (1000 tr/mn). On a ainsi obtenu un liquide blanc-jaune.

La composition appliquée sur le corps libère le parfum au cours de la journée au contact de la transpiration ou de l'humidité.

### Exemple 4 : Huile de massage

| **Ingrédients** | **(% en poids)** |
|---|---|
| Cyclopentadimethylsiloxane | 32 |
| Trigycerides caprylique/caprique | 42,95 |
| Palmitate d'isopropyle | 22 |
| Capsules de parfum de l'exemple H | 1 |
| Dimethicone / Trimethylsiloxy silicate | 2 |
| Ditertiobutyl 4-hydroxytoluène | 0,05 |
| Total | 100,00 |

Les capsules de parfum de l'exemple H peuvent être remplacées par les capsules des exemples A à G décrites précédemment

On a ajouté dans une cuve équipée d'un agitateur 11,0g de palmitate d'isopropyle 16,0g de cyclopenta dimethylsiloxane, 0,025g de ditertiobutyl hydroxytoluène, 21,475g de Triglycérides caprylique/caprique et 1,0g de dimethicone /trimethylsiloxy silicate. On a homogénéisé le mélange sous agitation vive (1000tr/mn). Puis on a ajouté 0,5g de capsules de l'exemple H. On a homogénéisé à nouveau le mélange sous agitation vive (1000 tr/mn). On a ainsi obtenu un liquide.

La composition appliquée sur le corps libère le parfum au cours du massage et au cours de la journée au contact de la transpiration ou de l'humidité.

### Exemple 5 : Huile de massage

De façon similaire on a préparé une huile de massage ayant la composition suivante :

| **Ingrédients** | **(% en poids)** |
|---|---|
| Cyclopentadimethylsiloxane | 32 |
| Trigycerides caprylique/caprique | 40,95 |
| Palmitate d'isopropyle | 22 |
| Capsules de parfum de l'exemple H | 1 |
| Dimethicone / Trimethylsiloxy silicate | 2 |
| Ditertiobutyl 4-hydroxytoluène | 0,05 |
| parfum | 2 |
| Total | 100,00 |

Les capsules de parfum de l'exemple H peuvent être remplacées par les capsules des exemples A à G décrites précédemment

La composition appliquée sur le corps libère le parfum au cours du massage et au cours de la journée au contact de la transpiration ou de l'humidité.

### Exemple 6 : huile de massage

De façon similaire on a préparé une huile de massage ayant la composition suivante

| **Ingrédients** | **(% en poids)** |
|---|---|
| Cyclopentadimethylsiloxane | 32 |
| Trigycerides caprylique/caprique | 40,95 |
| Palmitate d'isopropyle | 22 |
| Capsules de parfum de l'exemple A | 1 |
| Dimethicone / Trimethylsiloxy silicate | 2 |
| Ditertiobutyl 4-hydroxytoluène | 0,05 |
| parfum | 2 |
| Total | 100,00 |

Les capsules de parfum de l'exemple A peuvent être remplacées par les capsules des exemples B à H décrites précédemment

La composition appliquée sur le corps libère le parfum au cours de la journée au contact de la transpiration ou de l'humidité.

### Exemple 7 : Huile capillaire

De façon similaire, on a préparé une huile capillaire ayant la composition suivante :

| Ingrédients | % en poids |
|---|---|
| Huile de sésame | 15,0 |
| Huile d'amande douce | 12,1 |
| Isododécane | 14,3 |
| Methoxycinnamate d'éthylhexyle | 0,5 |
| Polyisobutène hydrogéné | 20,0 |
| Parfum | 3,0 |
| Palmitate d'isopropyle | 34,1 |
| Capsule de parfum de l'exemple A | 1,0 |

Les capsules de parfum de l'exemple A peuvent être remplacées par les capsules des exemples B à H décrites précédemment

Après l'application de la composition sur les cheveux. on constate lorsque le sujet transpire ou au contact du sébum ou de l'humidité qu'une libération du parfum se produit au cours de la journée.

## Revendications

1. Composition anhydre sous forme d'huile comprenant :
1) au moins des particules comprenant un cœur contenant au moins un agent bénéfique et une enveloppe entourant le cœur ; ladite enveloppe comprenant au moins un polysaccharide modifié hydrophobe choisi parmi les C₅-C₂₀-alcényl succinates d'amidon et au moins un glucide hydrosoluble choisi parmi les maltodextrines ayant un Dextrose Equivalent allant de 4 à 20;
lesdites particules présentant simultanément une densité de poudre versée allant de 300,0 g/l à 600,0 g/l et une densité absolue supérieure à 1.0 ;
lesdites particules étant sphériques et ayant un diamètre moyen en nombre allant de 1 à 30µm et un diamètre moyen en volume allant de 5 à 150µm ;
et
2) une phase huileuse.

2. Composition selon la revendication 1, comprenant un milieu physiologiquement acceptable.

3. Composition selon la revendication 1 ou 2, où les particules sont sphériques et en particulier ont un diamètre moyen en nombre allant de 2 à 15 µm et de préférence de 5 à 10 µm et un diamètre moyen en volume allant de 10 à 100 µm et de préférence de 20 à 80 µm.

4. Composition selon l'une quelconque des revendications 1 à 3, où le polysaccharide modifié hydrophobe l'octényle succinate d'amidon sodique.

5. Composition selon l'une quelconque des revendications 1 à 4, où le polysaccharide modifié hydrophobe représente de 20 à 90% en poids, notamment 30 à 80% en poids, mieux 40 à 70% en poids, et encore mieux 40 à 60% en poids par rapport au poids total de l'enveloppe de la particule.

6. Composition selon l'une quelconque des revendications précédentes, où le glucide hydrosoluble est choisi parmi les maltodextrines de D.E. allant de 12 à 20.

7. Composition selon l'une quelconque des revendications précédentes, où le ou les glucides (s) hydrosoluble(s) représentent de 10 à 80% en poids, de préférence de 15 à 70% en poids, plus préférentiellement de 20 à 65% en poids, et encore mieux de 40 à 60% en poids par rapport au poids total de l'enveloppe de la particule.

8. Composition selon l'une quelconque des revendications précédentes, où l'enveloppe des particules à libération d'agent bénéfique est constituée
a) d'au moins un C₅-C₂₀-alcenylsuccinate d'amidon dans une quantité allant de 20 à 90% en poids, notamment 30 à 80% en poids, mieux 40 à 70% en poids, et encore mieux 40 à 60% en poids par rapport au poids total de l'enveloppe de la particule et
b) moins une maltodextrine de D.E. allant de 4 à 20 et de préférence allant de 12 à 20, dans une quantité allant de 10 à 80% en poids, de préférence de 15 à 70% en poids, plus préférentiellement de 20 à 65% en poids, et encore mieux de 40 à 60% en poids par rapport au poids total de l'enveloppe de la particule.

9. Composition selon l'une quelconque des revendications précédentes, où les particules à libération d'agent bénéfique sont susceptibles d'être obtenues selon un procédé comprenant au moins les étapes suivantes :
- on prépare une solution aqueuse constituée du mélange du glucide hydrosoluble choisi parmi les maltodextrines ayant un Dextrose Equivalent allant de 4 à 20 et du polysaccharide modifié hydrophobe choisi parmi les C₅-C₂₀-alcényl succinates d'amidon puis on ajoute l'agent bénéfique et on agite de façon à former une émulsion ; et
- on homogénéise ladite émulsion ainsi formée sous haute pression à une pression allant de 10 à 200 bars et plus préférentiellement de 20 à 200 bars ; et
- on pulvérisée ladite émulsion dans une chambre de séchage, et
- l'eau est extraite pendant une durée de préférence ne dépassant pas 3 heures, et plus préférentiellement ne dépassant pas 30 minutes, avec un fluide sous pression tel que le dioxyde de carbone, de préférence à l'état supercritique de préférence à une pression d'au moins 0,3XPc et à une température d'au moins Tc-60°C avec Pc correspondant à la pression critique du gaz et Tc la température critique du gaz, de façon à obtenir les particules à libération d'agent bénéfique.

10. Composition selon l'une quelconque des revendications précédentes, où les agents bénéfiques sont choisis parmi :
(i) les corps gras ;
(ii) les substances parfumantes ;
(iii) les principes actifs pharmaceutiques ;
(iv) les actifs cosmétiques

11. Composition selon l'une quelconque des revendications précédentes, où les agents bénéfiques sont choisis parmi les substances parfumantes et plus particulièrement celles choisies parmi les notes de cœur et/ou de tête et encore plus particulièrement choisies parmi :
Acétate de benzyle
Acétate de géranyle
Acétate de cis-3-hexenyle
Aldéhyde C18 ou nonalactone
Acétate de décyle
Allyl amyl glycolate (citral)
Acétate d'éthyle
Acétate de butyle
Allyl 3-cyclohexylpropionate
Acétate de linalyle
Alcool Phényléthylique
Acétate d'héxyle
Berryflor ou éthyl-6-(acétyloxy)-hexanoate
Acétate d'isoamyle
Caproate d'allyle
Amarocite ou 6,6-diméthoxy-2,5,5-triméthylhex-2-ène
Citral lemarome N ou 3,7-diméthylocta-2,6-diénal
Canthoxal ou anisyl propanal
Claritone ou 2,4,4,7-tétraméthyloct-6-èn-3-one
Ethyl-2méthyl butyrate
Dihydromyrcénol
Cis-3 hexenol
Hédione ou méthyl dihydrojasmonate
L-carvone
Heptanoate d'allyle
Limonène
Néobuténone alpha ou 1-(5,5-diméthyl-1-cyclohexenyl)- pent-4-èn-1-one
Méthylheptènone
Toscanol ou 4-(cyclopropylméthyl)-phénylméthyl éther
myrcenol super ou 2-methyl-6-methylideneoct-7-en-2-ol
Decalactone
Acétate de stéaryle
Oxyde de rose
Linalool
Triplal ou 2,4-dimethylcyclohex-3-ène-1-carbaldéhyde
Mélonal ou 2,6-dimethylhept-5-ènal
1-(1,2,3,4,5,6,7,8-octahydro-2,3,8,8-tetraméethyl-2-naphthyl)éthan-1-one
Hexylcinnamal
Tétrahydro-2-isobutyl-4-méthylpyrann-4-ol
Salicylate d'hexyle
1,4-Dioxacycloheptadécane-5,17-dione
et leurs mélanges

12. Composition selon l'une quelconque des revendications précédentes, où les particules comprennent au moins une ou plusieurs substances parfumantes ayant une pression de vapeur saturante à 25°C supérieure ou égaie à 10,0 Pa et, préférentiellement, la ou lesdites substances parfumantes représentent de 50 à 100% en poids, de préférence de 60 à 100% en poids, plus préférentiellement de 70 à 100% en poids, et encore mieux de 80 à 100% en poids du poids total des substances parfumantes présentes dans les particules

13. Composition selon l'une quelconque des revendications précédentes, **caractérisée par le fait que**
a) les particules comprennent au moins une substance parfumante et
b) la composition comprend en plus au moins une substance parfumante sous forme libre, identique ou différente de la substance parfumante présente dans lesdites particules.

14. Composition selon l'une quelconque des revendications 1 à 12, **caractérisée par le fait qu'**elle contient exclusivement une ou plusieurs substances parfumantes encapsulées dans les particules.

15. Composition selon l'une quelconque des revendications précédentes, comprenant au moins un actif déodorant et/ou au moins un actif anti-transpirant et où, plus particulièrement, les particules à libération d'agent bénéfique comprennent au moins une substance parfumante, et encore plus particulièrement, où la composition comprend en plus au moins une substance parfumante sous forme libre, identique ou différente de la substance parfumante présente dans lesdites particules.

16. Procédé de soin et/ou de maquillage d'une matière kératinique humaine consistant à appliquer sur la surface de ladite matière kératinique humaine une composition selon l'une quelconque des revendications précédentes.

17. Procédé cosmétique de traitement des odeurs corporelles et éventuellement de la transpiration humaine consistant à appliquer sur la surface de la matière kératinique une composition selon la revendication 15.

18. Produit de consommation, **caractérisé par le fait qu'**il est constitué par une composition telle que définie selon l'une quelconque des revendications 1 à 15.

## Patentansprüche

1. Wasserfreie Zusammensetzung in Ölform, umfassend
1) mindestens Teilchen mit einem Kern, der mindestens einen Effektstoff enthält, und einer den Kern umgebenden Hülle; wobei die Hülle mindestens ein hydrophob modifiziertes Polysaccharid, das aus Stärke-C₅-C₂₀-alkenylsuccinaten ausgewählt ist, und mindestens ein wasserlösliches Kohlenhydrat, das aus Maltodextrinen mit einem Dextroseäquivalent im Bereich von 4 bis 20 ausgewählt ist, umfasst;
wobei die Teilchen gleichzeitig eine Pulverschüttdichte im Bereich von 300,0 g/l bis 600,0 g/l und eine absolute Dichte von mehr als 1,0 aufweisen; wobei die Teilchen kugelförmig sind und einen zahlenmittleren Durchmesser im Bereich von 1 bis 30 µm und einen volumenmittleren Durchmesser im Bereich von 5 bis 150 µm aufweisen;
und
2) eine ölige Phase.

2. Zusammensetzung nach Anspruch 1, umfassend ein physiologisch unbedenkliches Medium.

3. Zusammensetzung nach Anspruch 1 oder 2, wobei die Teilchen kugelförmig sind und insbesondere einen zahlenmittleren Durchmesser im Bereich von 2 bis 15 µm und vorzugsweise von 5 bis 10 µm und einen volumenmittleren Durchmesser im Bereich von 10 bis 100 µm und vorzugsweise von 20 bis 80 µm aufweisen.

4. Zusammensetzung nach einem der Ansprüche 1 bis 3, wobei es sich bei dem hydrophob modifizierten Polysaccharid um Natriumstärkeoctenylsuccinat handelt.

5. Zusammensetzung nach einem der Ansprüche 1 bis 4, wobei das hydrophob modifizierte Polysaccharid 20 bis 90 Gew.-%, insbesondere 30 bis 80 Gew.-%, besser 40 bis 70 Gew.-% und noch besser 40 bis 60 Gew.-%, bezogen auf das Gesamtgewicht der Hülle des Teilchens, ausmacht.

6. Zusammensetzung nach einem der vorhergehenden Ansprüche, wobei das wasserlösliche Kohlenhydrat aus Maltodextrinen mit einem D.E. im Bereich von 12 bis 20 ausgewählt ist.

7. Zusammensetzung nach einem der vorhergehenden Ansprüche, wobei das wasserlösliche Kohlenhydrat bzw. die wasserlöslichen Kohlenhydrate 10 bis 80 Gew.-%, vorzugsweise 15 bis 70 Gew.-%, vorzugsweise 20 bis 65 Gew.-% und noch besser 40 bis 60 Gew.-%, bezogen auf das Gesamtgewicht der Hülle des Teilchens, ausmacht bzw. ausmachen.

8. Zusammensetzung nach einem der vorhergehenden Ansprüche, wobei die Hülle der Teilchen mit Freisetzung von Effektstoff aus
a) mindestens einem Stärke-C₅-C₂₀-alkenylsuccinat in einer Menge im Bereich von 20 bis 90 Gew.-%, insbesondere 30 bis 80 Gew.-%, besser 40 bis 70 Gew.-% und noch besser 40 bis 60 Gew.-%, bezogen auf das Gesamtgewicht der Hülle des Teilchens, und
b) mindestens einem Maltodextrin mit einem D.E. im Bereich von 4 bis 20 und vorzugsweise im Bereich von 12 bis 20 in einer Menge im Bereich von 10 bis 80 Gew.-%, vorzugsweise von 15 bis 70 Gew.-%, weiter bevorzugt von 20 bis 65 Gew.-% und noch besser von 40 bis 60 Gew.-%, bezogen auf das Gesamtgewicht der Hülle des Teilchens,
besteht.

9. Zusammensetzung nach einem der vorhergehenden Ansprüche, wobei die Teilchen mit Freisetzung von Effektstoff gemäß einem Verfahren erhältlich sind, das mindestens die folgenden Schritte umfasst:
- man stellt eine wässrige Lösung her, die aus der Mischung des wasserlöslichen Kohlenhydrats, das aus Maltodextrinen mit einem Dextroseäquivalent im Bereich von 4 bis 20 ausgewählt ist, und des hydrophob modifizierten Polysaccharids, das aus Stärke-C₅-C₂₀-alkenylsuccinaten ausgewählt ist, besteht, gibt dann den Effektstoff zu und rührt zur Bildung einer Emulsion; und
- man homogenisiert die so gebildete Emulsion unter hohem Druck bei einem Druck im Bereich von 10 bis 200 bar und weiter bevorzugt von 20 bis 200 bar; und
- man versprüht die Emulsion in einer Trocknungskammer; und
- man extrahiert das Wasser über einen Zeitraum, der vorzugsweise nicht mehr als 3 Stunden und weiter bevorzugt nicht mehr als 30 Minuten beträgt, mit einem unter Druck stehenden Fluid wie Kohlendioxid, vorzugsweise in überkritischem Zustand, vorzugsweise bei einem Druck von mindestens 0,3xPc und einer Temperatur von mindestens Tc-60 °C, wobei Pc dem kritischen Druck des Gases entspricht und Tc der kritischen Temperatur des Gases entspricht, wodurch man die Teilchen mit Freisetzung von Effektstoff erhält.

10. Zusammensetzung nach einem der vorhergehenden Ansprüche, wobei die Effektstoffe aus
(i) Fettsubstanzen;
(ii) Parfümierungssubstanzen;
(iii) pharmazeutischen Wirkstoffen;
(iv) kosmetischen Wirkstoffen
ausgewählt sind.

11. Zusammensetzung nach einem der vorhergehenden Ansprüche, wobei die Effektstoffe aus Parfümierungssubstanzen ausgewählt sind und spezieller aus jenen, die aus Herz- und/oder Kopfnoten und noch spezieller aus
Benzylacetat
Geranylacetat
cis-3-Hexenylacetat
C₁₈-Aldehyd oder Nonalacton
Decylacetat
Allylamylglykolat (Citral)
Ethylacetat
Butylacetat
Allyl-3-cyclohexylpropionat
Linalylacetat
Phenylethylalkohol
Hexylacetat
Berryflor oder Ethyl-6-(acetyloxy)hexanoat
Isoamylacetat
Allylcaproat
Amarocite oder 6,6-Dimethoxy-2,5,5-trimethylhex-2-en
Citral lemarome N oder 3,7-Dimethylocta-2,6-dienal Canthoxal oder Anisylpropanal
Claritone oder 2,4,4,7-Tetramethyloct-6-en-3-on Ethyl-2-methylbutyrat
Dihydromyrcenol
cis-3-Hexenol
Hedion oder Methyldihydrojasmonat
L-Carvon
Allylheptanoat
Limonen
Neobutenon alpha oder 1-(5,5-Dimethyl-1-cyclohexenyl)pent-4-en-1-on
Methylheptenon
Toscanol oder 4-(Cyclopropylmethyl)phenylmethyl-ether
Myrcenol super oder 2-Methyl-6-methylidenoct-7-en-2-ol
Decalacton
Stearylacetat
Rosenoxid
Linalool
Triplal oder 2,4-Dimethylcyclohex-3-en-1-carbaldehyd
Melonal oder 2,6-Dimethylhept-5-enal
1-(1,2,3,4,5,6,7,8-Octahydro-2,3,8,8-tetramethyl-2-naphthyl)ethan-1-on
Hexylcinnamal
Tetrahydro-2-isobutyl-4-methylpyran-4-ol
Hexylsalicylat
1,4-Dioxacycloheptadecan-5,17-dion
und Mischungen davon
ausgewählt sind.

12. Zusammensetzung nach einem der vorhergehenden Ansprüche, wobei die Teilchen mindestens eine oder mehrere Parfümierungssubstanzen mit einem Sättigungsdampfdruck bei 25 °C größer oder gleich 10,0 Pa aufweisen und die Parfümierungssubstanz bzw. die Parfümierungssubstanzen vorzugsweise 50 bis 100 Gew.-%, weiter bevorzugt 60 bis 100 Gew.-%, noch weiter bevorzugt 70 bis 100 Gew.-% und noch besser 80 bis 100 Gew.-% des Gesamtgewichts der in den Teilchen vorliegenden Parfümierungssubstanzen ausmacht bzw. ausmachen.

13. Zusammensetzung nach einem der vorhergehenden Ansprüche, **dadurch gekennzeichnet, dass**
a) die Teilchen mindestens eine Parfümierungssubstanz umfassen und
b) die Zusammensetzung außerdem mindestens eine Parfümierungssubstanz in freier Form, die mit der in den Teilchen vorliegenden Parfümierungssubstanz identisch oder davon verschieden ist, umfasst.

14. Zusammensetzung nach einem der Ansprüche 1 bis 12, **dadurch gekennzeichnet, dass** sie ausschließlich eine oder mehrere in den Teilchen verkapselte Parfümierungssubstanzen enthält.

15. Zusammensetzung nach einem der vorhergehenden Ansprüche, umfassend mindestens einen Deodorantwirkstoff und/oder mindestens einen Antitranspirantwirkstoff, und/oder spezieller wobei die Teilchen mit Freisetzung von Effektstoff mindestens eine Parfümierungssubstanz umfassen und noch spezieller wobei die Zusammensetzung außerdem mindestens eine Parfümierungssubstanz in freier Form, die mit der in den Teilchen vorliegenden Parfümierungssubstanz identisch oder davon verschieden ist, umfasst.

16. Verfahren zur Pflege und/oder zum Schminken eines menschlichen Keratinmaterials, das darin besteht, dass man auf das menschliche Keratinmaterial eine Zusammensetzung nach einem der vorhergehenden Ansprüche aufbringt.

17. Kosmetisches Verfahren zur Behandlung von Körpergerüchen und gegebenenfalls menschlicher Transpiration, das darin besteht, dass man eine Zusammensetzung nach Anspruch 15 auf die Oberfläche des Keratinmaterials aufbringt.

18. Konsumprödukt, **dadurch gekennzeichnet, dass** es aus einer Zusammensetzung nach einem der Ansprüche 1 bis 15 besteht.

## Claims

1. Anhydrous composition in oil form comprising:
1) at least particles comprising a core containing at least one beneficial agent and a shell surrounding the core; the said shell comprising at least one hydrophobic-modified polysaccharide chosen from starch C₅-C₂₀-alkenyl succinates and at least one water-soluble carbohydrate chosen from maltodextrins having a Dextrose Equivalent ranging from 4 to 20;
the said particles simultaneously exhibiting a poured powder density ranging from 300.0 g/l to 600.0 g/l and an absolute density of greater than 1.0;
the said particles being spherical and having a number-average diameter ranging from 1 to 30 microns and a volume-average diameter ranging from 5 to 150 microns;
and
2) an oily phase.

2. Composition according to Claim 1, comprising a physiologically acceptable medium.

3. Composition according to Claim 1 or 2, where the particles are spherical and in particular have a number-average diameter ranging from 2 to 15 microns and preferably from 5 to 10 microns and a volume-average diameter ranging from 10 to 100 microns and preferably from 20 to 80 microns.

4. Composition according to any one of Claims 1 to 3, where the hydrophobic-modified polysaccharide is starch sodium octenyl succinate.

5. Composition according to any one of Claims 1 to 4, where the hydrophobic-modified polysaccharide represents from 20% to 90% by weight, in particular from 30% to 80% by weight, better still from 40% to 70% by weight and even better still from 40% to 60% by weight, with respect to the total weight of the shell of the particle.

6. Composition according to any one of the preceding claims, where the water-soluble carbohydrate is chosen from maltodextrins with a DE ranging from 12 to 20.

7. Composition according to any one of the preceding claims, where the water-soluble carbohydrate(s) represent from 10% to 80% by weight, preferably from 15% to 70% by weight, more preferentially from 20% to 65% by weight and better still from 40% to 60% by weight, with respect to the total weight of the shell of the particle.

8. Composition according to any one of the preceding claims, where the shell of the particles having release of beneficial agent is constituted
a) of at least one starch C₅-C₂₀ alkenyl succinate in an amount ranging from 20% to 90% by weight, in particular from 30% to 80% by weight, better still from 40% to 70% by weight and even better still from 40% to 60% by weight, with respect to the total weight of the shell of the particle, and
b) of at least one maltodextrin with a DE ranging from 4 to 20 and preferably ranging from 12 to 20, in an amount ranging from 10% to 80% by weight, preferably from 15% to 70% by weight, more preferentially from 20% to 65% by weight and better still from 40% to 60% by weight, with respect to the total weight of the shell of the particle.

9. Composition according to any one of the preceding claims, where the particles having release of beneficial agents are capable of being obtained according to a process comprising at least the following stages:
- an aqueous solution constituted of the mixture of the water-soluble carbohydrate chosen from maltodextrins having a Dextrose Equivalent ranging from 4 to 20 and hydrophobic-modified polysaccharide chosen from starch C₅-C₂₀-alkenyl succinates is prepared, the beneficial agent is then added and stirring is carried out so as to form an emulsion; and
- the said emulsion thus formed is homogenized under high pressure at a pressure ranging from 10 to 200 bar and more preferentially from 20 to 200 bar; and
- the said emulsion is sprayed into a drying chamber; and
- the water is extracted over a period of time preferably not exceeding 3 hours and more preferentially not exceeding 30 minutes, with a pressurized fluid, such as carbon dioxide, preferably in the supercritical state, preferably at a pressure of at least 0.3 × Pc and at a temperature of at least Tc-60°C, with PC corresponding to the critical pressure of the gas and Tc corresponding to the critical temperature of the gas, so as to obtain the particles having release of beneficial agent.

10. Composition according to any one of the preceding claims, where the beneficial agents are chosen from:
(i) fatty substances;
(ii) fragrancing substances;
(iii) pharmaceutical active principles;
(iv) cosmetic active principles.

11. Composition according to any one of the preceding claims, where the beneficial agents are chosen from fragrancing substances and more particularly those chosen from the heart and/or top notes and more particularly still chosen from:
Benzyl acetate
Geranyl acetate
cis-3-Hexenyl acetate
C₁₈ aldehyde or nonalactone
Decyl acetate
Allyl amyl glycolate (citral)
Ethyl acetate
Butyl acetate
Allyl 3-cyclohexylpropionate
Linalyl acetate
Phenylethyl alcohol
Hexyl acetate
Berryflor or ethyl 6-(acetyloxy)hexanoate
Isoamyl acetate
Allyl caproate
Amarocite or 6,6-dimethoxy-2,5,5-trimethylhex-2-ene
Citral lemarome N or 3,7-dimethylocta-2,6-dienal
Canthoxal or anisyl propanal
Claritone or 2,4,4,7-tetramethyloct-6-en-3-one
Ethyl 2-methylbutyrate
Dihydromyrcenol
cis-3-Hexenol
Hedione or methyl dihydrojasmonate
L-Carvone
Allyl heptanoate
Limonene
Neobutenone alpha or 1-(5,5-dimethyl-1-cyclohexenyl)pent-4-en-1-one
Methylheptenone
Toscanol or 4-(cyclopropylmethyl)phenyl methyl ether
Myrcenol Super or 2-methyl-6-methylideneoct-7-en-2-ol
Decalactone
Stearyl acetate
Rose oxide
Linalool
Triplal or 2,4-dimethylcyclohex-3-ene-1-carbaldehyde Melonal or 2,6-dimethylhept-5-enal
1-(1,2,3,4,5,6,7,8-Octahydro-2,3,8,8-tetramethyl-2-naphthyl)ethan-1-one
Hexylcinnamal
Tetrahydro-2-isobutyl-4-methylpyran-4-ol Hexyl salicylate
1,4-Dioxacycloheptadecane-5,17-dione
and their mixtures.

12. Composition according to any one of the preceding claims, where the particles comprise at least one or more fragrancing substances having a saturated vapour pressure at 25°C of greater than or equal to 10.0 Pa and preferentially the said fragrancing substance(s) represent from 50% to 100% by weight, preferably from 60% to 100% by weight, more preferentially from 70% to 100% by weight and better still from 80% to 100% by weight of the total weight of the fragrancing substances present in the particles.

13. Composition according to any one of the preceding claims, **characterized in that**
a) the particles comprise at least one fragrancing substance and
b) the composition additionally comprises at least one fragrancing substance in the free form, identical to or different from the fragrancing substance present in the said particles.

14. Composition according to any one of Claims 1 to 12, **characterized in that** it exclusively contains one or more fragrancing substances encapsulated in the particles.

15. Composition according to any one of the preceding claims, comprising at least one deodorant active principle and/or at least one antiperspirant active principle and where more particularly the particles having release of beneficial agent comprise at least one fragrancing substance and more particularly still where the composition additionally comprises at least one fragrancing substance in the free form, identical to or different from the fragrancing substance present in the said particles.

16. Method for caring for and/or making up a human keratinous substance which consists in applying, to the surface of said human keratinous substance, a composition according to any one of the preceding claims.

17. Cosmetic method for the treatment of body odours and optionally of perspiration of human beings which consists in applying, to the surface of the keratinous substance, a composition according to Claim 15.

18. Consumer product, **characterized in that** it is constituted of a composition as defined according to any one of Claims 1 to 15.
